# EUROPEAN PATENT SPECIFICATION

(11) **EP 1 694 851 B1**
(45) Date of publication and mention of the grant of the patent: **05.05.2010**
(21) Application number: 04801361.9
(22) Date of filing: 08.12.2004
(51) Int. Cl.: C12N 15/86

(54) **VIRAL VECTORS CONTROLLED BY A GENE SWITCH**
GENSCHALTER-KONTROLLIERTE VIRALE VEKTOREN
VECTEURS VIRAUX REGULES PAR UNE COMMUTATION GENIQUE

(30) Priority: 10.12.2003 US 731961
(43) Date of publication of application: 30.08.2006
(73) Proprietor: HSF Pharmaceuticals, S.A., CH-1814 La Tour-de-Peilz (CH)
(72) Inventor: VOELLMY, Richard, CH-1814 La Tour-de-Peilz (CH)
(74) Representative: Völlmy, Lukas
(86) International application number: PCT/IB2004/004067
(87) International publication number: WO 2005/056806

(56) References cited:
- WO-A-99/57290
- WO-A-02/090501
- CHONG HEUNG ET AL: "A system for small-molecule control of conditionally replication-competent adenoviral vectors." MOLECULAR THERAPY : THE JOURNAL OF THE AMERICAN SOCIETY OF GENE THERAPY. FEB 2002, vol. 5, no. 2, February 2002 (2002-02), pages 195-203, XP002344532 ISSN: 1525-0016
- XU Z-L ET AL: "Regulated gene expression from adenovirus vectors: a systematic comparison of various inducible systems" GENE: AN INTERNATIONAL JOURNAL ON GENES AND GENOMES, ELSEVIER SCIENCE PUBLISHERS, BARKING, GB, vol. 309, no. 2, 8 May 2003 (2003-05-08), pages 145-151, XP004426691 ISSN: 0378-1119
- SMITH ROY C ET AL: "Spatial and temporal control of transgene expression through ultrasound-mediated induction of the heat shock protein 70B promoter in vivo" HUMAN GENE THERAPY, vol. 13, no. 6, 10 April 2002 (2002-04-10), pages 697-706, XP002344533 ISSN: 1043-0342
- POST DAWN E ET AL: "Replicative oncolytic adenoviruses in multimodal cancer regimens." HUMAN GENE THERAPY, vol. 14, no. 10, 1 July 2003 (2003-07-01), pages 933-946, XP002344534 ISSN: 1043-0342 cited in the application
- VILABOA N ET AL: "Novel Gene Switches for Targeted and Timed Expression of Proteins of Interest" MOLECULAR THERAPY, ACADEMIC PRESS, SAN DIEGO, CA,, US, vol. 12, no. 2, August 2005 (2005-08), pages 290-298, XP004991975 ISSN: 1525-0016

## Description

### TECHNICAL FIELD

The present invention relates to viral vectors whose replication and, optionally, passenger gene expression are regulated by a gene switch that is dually controlled by heat and a small molecule regulator.

### BACKGROUND OF THE INVENTION

There are numerous situations in biological research, ex vivo cell therapies and gene therapies of experimental animals and, eventually, humans, where careful control of the distribution and expression of an introduced gene or a replicating virus is critically important. Viral and non-viral vectors provide means for delivering genes to cells, tissues and organs. However, this delivery is, typically, not specific for any particular cell type, tissue or organ location. Previously, so-called tissue-specific promoters were used to restrict virus replication or expression of a gene delivered by a vector to cells of a specific type. A shortcoming of this approach is that is uncertain whether a "tissue-specific" promoter really exists. Although a promoter may have a much stronger activity in a particular cell type than in others, it is likely to display at least some activity in some other cells. Furthermore, the rate of transcription supported by a chosen "tissue-specific" promoter, which rate is an intrinsic property of the promoter, may limit its usefulness. Other approaches for controll ing expression of therapeutic genes included the use of two-component gene switches that are activated or inactivated by a small molecule regulator. Well-known switches are controlled by tetracycline, ecdysterone, mifepristone or rapamycin. While these switches may allow for stringent on-off regulation of gene activity, basal gene activity cannot be controlled and intermediate levels of gene activity are typically difficult to achieve. Moreover, gene expression cannot be locally restricted by a small molecule regulator that readily diffuses through tissues. Because an activating heat treatment can be directed to a target tissue, heat shock gene promoters should provide an effective means for restricting expression of a gene delivered by a viral or other vector to a target tissue. These promoters have also been suggested to be useful for controlling viral replication (U.S. 09/850270). Some of these promoters, in particular a human hsp70B gene promoter isolated and characterized by the inventor, have minimal basal activity and a heat-induced activity that can be a thousand fold greater than basal activity. Such promoters should be particularly useful for achieving strict regulation of localized gene expression or virus replication. However, there is a downside to using promoters that are activated by transient heat and other proteotoxic stresses. After introduction into an experimental animal or a human, inadvertent activation of these promoters can occur during a fever or ischemia/reperfusion, exposure to oxidant or other proteotoxic agent, or, possibly, even hormonal stress. The confounding effects of such inadvertent, non-directed activation of virus replication or gene expression will be unacceptable in many applications. Gene switches that are only activated by a combination of heat (or other proteotoxic stress) and a small molecule regulator were previously described and were suggested to be useful to achieve spatial as well as temporal control of therapeutic gene expression (U.S. 6,342,596; U.S. patent application 10/046420).

### SUMMARY OF THE INVENTION

The present invention relates to a modified, conditionally replication-competent virus whose genome includes a gene switch activatable in an infected cell by exposure of the cell to heat and a small molecule regulator, the gene switch controlling the expression of a gene for a viral protein required for efficient replication of the modified virus. The invention similarly relates to a modified, conditionally replication-competent virus whose genome includes a gene switch that is activated in an infected cell by exposure of the cell to heat and is repressed by exposure of the cell to a small molecule regulator, the gene switch controlling the expression of a gene for a viral protein required for efficient replication of the modified virus. A modified, conditionally replication-competent virus of the invention may further include a passenger gene. Expression of the passenger gene can also be regulated by the gene switch that controls viral replication. Preferably, a modified, conditionally replication-competent virus is derived from a member of the Adenoviridae, Herpesviridae, and Retroviridae families. Most preferably, a modified, conditionally replication-competent virus is derived from a member of the Adenoviridae family. If the modified, conditionally replication-competent virus is an adenovirus, the genes that can be controlled by the gene switch include the E1A, E1B and E4 genes. The gene switch that controls the expression of one or more viral genes, whose product is required for or facilitates viral replication, comprises two components. The first component is a gene for a transactivator that is activated or inhibited by a small molecule regulator. In preferred embodiments, expression of the transactivator gene is controlled by a heat shock promoter activatable by transient heat or a transient proteotoxic stress through the intermediary of endogenous heat shock factor 1 (HSF1), or by a tandem or hybrid promoter activatable by transient heat or proteotoxic stress through the intermediary of endogenous HSF1 and by active transactivator, The second component of the switch is a promoter activated by the active form of the transactivator, which promoter is functionally linked to a viral or passenger gene to be regulated. In alternative embodiments, the first component is a transactivator gene that is expressed continuously (constitutively) in a host cell. The second component can be a modified heat shock promoter (including an appropriate RNA leader region) that is activated by transient heat or other proteotoxic stress and repressed by the transactivator. Binding of a small molecule regulator to the transactivator can, respectively, inhibit or enable its repressing function. Hence, the resulting gene switch is active in cells exposed to heat or proteotoxic stress in the presence or absence, respectively, of the small molecule regulator. The second component can also be a modified (or partial) heat shock promoter that requires co-activation by transactivator and endogenous HSF1. In this case, transactivator is activated by a bound small molecule regulator.

The invention also relates to a pair of modified viruses whose combined genomes contain all genetic information required for conditional replication of the virus pair; including a gene switch activatable in an infected cell by exposure of the cell to heat and a small molecule regulator, the gene switch controlling the expression of a gene for a viral protein required for efficient replication of the virus pair. The invention further concerns a pair of modified viruses whose combined genomes contain all genetic information required for conditional replication of the virus pair; including a gene switch that is activated in an infected cell by exposure of the cell to heat and is repressed by exposure of the cell to a small molecule regulator, the gene switch controlling the expression of a gene for a viral protein required for efficient replication of the virus pair. A pair of modified viruses of the invention may further include a passenger gene that is inserted into the genome of one or the other virus of the pair. The passenger gene can also be controlled by the gene switch that controls viral replication. Preferably, the modified viruses of a pair are modified members of the Adenoviridae, Herpesviridae, and Retroviridae families. Most preferably, each virus of a pair is derived from a member of the Adenoviridae family. If both viruses of a pair of the invention are modified adenoviruses, the genes that can be controlled by the gene switch include the E1A, E1B and E4 genes. The gene switch that controls the expression of one or more viral genes, whose product is required for or facilitates viral replication, comprises two components. The first component is a gene for a transactivator that is activated or inhibited by a small molecule regulator. In preferred embodiments, expression of the transactivator gene is controlled by a heat shock promoter activatable by transient heat or a transient proteotoxic stress through the intermediary of endogenous heat shock factor 1 (HSF1), or by a tandem or hybrid promoter activatable by transient heat or proteotoxic stress through the intermediary of endogenous HSF1 and by active transactivator, The second component of the switch is a promoter activated by the active form of the transactivator, which promoter is functionally linked to a viral or passenger gene to be regulated. In alternative embodiments, the first component is a transactivator gene that is expressed continuously (constitutively) in a host cell. The second component can be a modified heat shock promoter (including an appropriate RNA leader region) that is activated by transient heat or other proteotoxic stress and repressed by the transactivator. Binding of a small molecule regulator to the transactivator can, respectively, inhibit or enable its repressing function. Hence, the resulting gene switch is active in cells exposed to heat or proteotoxic stress in the presence or absence, respectively, of the small molecule regulator. The second component can also be a modified (or partial) heat shock promoter that requires co-activation by transactivator and endogenous HSF1. In this case, transactivator is activated by a bound small molecule regulator.

### BRIEF DESCRIPTION OF FIGURES

Figure 1 presents a generic gene switch that can be activated by transient heat or proteotoxic stress in the absence or presence of a small molecule regulator.
Figure 2 provides an example of the switch of Figure 1, in which switch generic transactivator is replaced with mifepristone-activated activator GLVP.
Figure 3 outlines how the switches of Figures 1 and 2 can be incorporated in the genome of a single adenovirus or the genomes of a pair of adenoviruses, respectively.
Figure 4A presents a generic gene switch that can be activated by transient heat or proteotoxic stress in the absence or presence of a small molecule regulator. The switch contains an auto-activation loop that causes transactivator expression to be maintained subsequent transient activation. Part B of the Figure shows an example switch, in which generic transactivator is replaced with mifepristone-activated activator GLVP.
Figure 5 outlines how an example switch of Figure 4 can be incorporated in the genome of a single adenovirus or the genomes of a pair of adenoviruses, respectively. Part C of the Figure also illustrates how a passenger gene can be introduced.
Figure 6 generically outlines two types of alternative gene switches that can or may be used to regulate replication of viruses of the invention as well as passenger gene expression.

### BRIEF DESCRIPTION OF THE SEQUENCE LISTING

SEQ ID NO 1 provides the nucleotide sequence of pShuttle.
SEQ ID NO 2 provides the nucleotide sequence of pGene/V5-His.
SEQ ID NO 3 provides the nucleotide sequence of pXC1.
SEQ ID NO 4 provides the nucleotide sequence of pSwitch.

### DETAILED DESCRIPTION

Unless otherwise defined, all terms shall have their ordinary meaning in the relevant art. The following terms are defined:

"Replication of virus" or "virus/viral replication" is understood to mean multiplication of viral particles.

"Proteotoxic stress" is a physical or chemical insult that results in increased protein unfolding, reduces maturation of newly synthesized polypeptides or causes synthesis of proteins that are unable to fold properly.

A "small molecule regulator" is understood to be a low molecular weight ligand of a transactivator used in connection with this invention. Depending on the transactivator concerned, the small molecule regulator can inhibit or activate the transactivator. Small molecule regulator is typically, but not necessarily, smaller than 1000 Dalton (1 kDa).

To be able to use a single term for all regulatory proteins used in gene switches described herein, "transactivator" shall be understood hereinafter as being a DNA-binding protein, whose binding to a specific DNA sequence can affect, positively or negatively, transcription of a nearby gene. This definition includes traditional transactivators as well as proteins that interfere with transcription.

"Activated" when used in connection with a transactivated gene means that the rate of expression of the gene is at least one order of magnitude greater after activation than before activation. When used in connection with a transactivator, "active" or "activated" refers to a DNA-binding and transactivation-competent form of a transactivator in the case of a positively acting transactivator, and to a DNA-binding form of a transactivator in the case of a negatively acting transactivator.

"Promoter of a heat shock gene", "heat shock gene promoter" and "heat shock promoter" are used synonymously.

Herein, a virus, whose genome includes a non-viral gene, is either referred to as a "virus" or a "viral vector".

"Recombinant" refers to the presence of an alteration caused by an experimenter.

Preferred viruses of the present invention possess the engineered property that they only replicate when cells containing them are exposed to heat or another proteotoxic stress in the presence of a small molecule regulator. Replication of other preferred viruses can be triggered by heat or other proteotoxic stress in the absence of a small molecule regulator that represses replication. The viruses can further contain a passenger gene that may or may not be activated under the same conditions. Because heat can be directed, systemically present viruses of the invention can be activated locally, i.e., in a spatially restricted manner. The viruses of the invention may be used for research purposes. They may be introduced into the germline of a non-human animal or may be introduced systemically or locally at any stage of development, including adult stage. After administration or withdrawal, respectively, of small molecule regulator, the experimental animal may be subjected to one or more courses of localized heat to activate virus in a particular tissue or group of cells, resulting in the ablation of the targeted cells and cells in surrounding tissue and/or in activation and local spreading of an activated passenger gene and, consequently, of the product of the passenger gene. Such experiments can assist in defining the function of particular tissues or groups of cells in an adult or developing animal or in elucidating the biological or therapeutic consequences of local expression of a passenger gene. The viruses of the invention will also be useful in cell-based therapies. Cells of heterologous origin may provoke a graft-vs-host response, in spite of immunosuppressive therapy. Conditionally replicating viruses present in these cells may be specifically activated in a tissue in which rejection is observed to achieve localized killing of offending cells while maintaining the benefit of the therapy in tissues in which no rejection is observed. In other applications, immune cells containing conditionally replicating virus of the invention may be administered as a tumor therapy. Immune cells reaching the tumor may be locally activated by heat in the systemic presence (or absence) of a small molecule regulator to trigger virus replication and cell lysis in the tumor tissue. Local cell lysis will provoke a local inflammatory response that should enhance development of specific immunity. Alternatively, cells may be infected with virus of the invention also including a therapeutic gene. After localized or systemic administration of the cells, virus replication may be activated in a desired tissue location to trigger virus replication and local dissemination of a therapeutic gene, avoiding, e.g., toxicity associated with systemic expression of the therapeutic protein. The viruses of the invention may also be used in experimental gene therapy using model animals and, eventually, humans. For example, a virus of the invention may be introduced into a tumor. Heat treatment in the systemic presence/absence of a small molecule regulator will initiate virus replication and tumor cell killing. The therapeutic efficacy of virus of the invention may be enhanced by the presence of a therapeutic gene, e.g., a passenger gene encoding a cytotoxic protein. Such virus will not only be induced to replicate in tumor tissue but also to express the toxic protein, resulting in increased anti-tumor activity. Alternatively, a virus of the invention may not by itself kill cells but may include a therapeutic gene, e.g., a passenger gene encoding a cytotoxic protein. Localized replication of the virus in a tumor will result in the dissemination of virus and therapeutic gene throughout the tumor. Tumor cells will be killed by the action of the therapeutic protein.

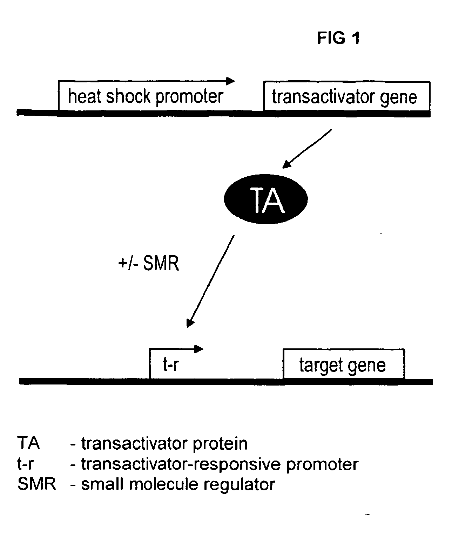

Replication of a virus of the invention is controlled by a gene switch that is activated by heat or other proteotoxic stress and the specific absence or presence of a small molecule regulator. Gene switches employed in the invention contain rivo components. Preferred gene switches used in connection with the conditionally replicating viruses of the invention comprise a gene for a small molecule-activated or small molecule-inhibited transactivator that is functionally linked to a promoter from a heat shock gene, and a promoter that is responsive to the transactivator. A gene to be regulated by the switch is functionally linked to the latter promoter that is responsive to the transactivator. In the case of adenovirus, the latter target gene may be the E1 gene. The operation of such a gene switch is outlined in Figure 1.

A heat shock gene is defined herein as any gene, from any eukaryotic organism, whose activity is substantially enhanced when the cell containing the gene is exposed to a temperature above its normal growth temperature. Typically, the genes are activated when ambient temperature is raised by 3-10°C. Heat shock genes comprise genes for the "classical" heat shock proteins, i.e., Hsp110, Hsp90, Hsp70, Hsp60, Hsp40, and Hsp20-30. They also include other heat-inducible genes such as genes for MDR1, ubiquitin, FKBP52, hemoxidase and other proteins. The promoters of these genes, the "heat shock promoters", contain characteristic sequence elements referred to as heat shock elements (HSE) that consist of perfect or imperfect sequence modules of the type NGAAN or AGAAN, which modules are arranged in alternating orientations (Xiao, H, and Lis, J.T. 1988. Science 239, 1139-1142; Amin, J., Ananthan, J., and Voellmy, R. 1988. Mol. Cell. Biol 8, 3761-3769; Fernandes, M., Xiao, H., and Lis, J.T. 1994. Nucleic Acids Res. 22, 167-173). These elements are highly conserved in all eukaryotic cells such that, e.g., a heat shock promoter from a fruit fly is functional and heat-regulated in a frog cell (Voellmy, R., and Rungger, D. 1982. Proc. Natl. Acad. Sci. USA 79, 1776-1780). HSE sequences are binding sites for heat shock transcription factors (HSFs; reviewed in Wu, C. 1995. Annu. Rev. Cell Dev. Biol. 11, 441-469). The factor responsible for activation of heat shock genes in vertebrate cells exposed to heat or a proteotoxic stress is HSF1 (Baler, R., Dahl, G., and Voellmy, R. 1993. Mol. Cell. Biol. 13, 2486-2496; McMillan, D.R., Xiao, X., Shao, L., Graves, K., and Benjamin, I.J. 1998. J. Biol. Chem. 273, 7523-7528). Preferred promoters are those from inducible hsp70 genes. A particularly preferred heat shock promoter used in the viruses of the present invention is the promoter of the human hsp70B gene (Voellmy, R., Ahmed, A., Schiller, P., Bromley, P., and Rungger, D. 1985. Proc. Natl. Acad. Sci. USA 82, 4949-4953).

Examples for small molecule-regulated transactivators than can be incorporated in the gene switches used in viruses of the invention include tetracycline/doxocycline-regulated tet-on and tet-off repressors (Gossen, M. and Bujard, H. 1992. Proc. Natl. Acad. Sci. USA 89, 5547-5551; Gossen, M., Freundlieb, S., Bender, G., Muller, G., Hillen, W. and Bujard, H. 1996. Science 268, 1766-1769), ecdysterone/ponasterone-regulated insect steroid receptor-based transcription factor EcR/RXR (No, D., Yao, T.P., and Evans, R.M. 1996. Proc. Natl. Acad. Sci. USA 93, 3346-3351), mifepristone-regulated GAL4-steroid receptor-activation domain chimeras such as GLVP (Wang, Y., DeMayo, F.J., Tsai, S.Y., and O'Malley, B.W. 1997. Nat. Biotechnol. 15, 239-243; Wang, Y., Xu, J., Pierson, T., O'Malley, B.W., and Tsai, S.Y. 1997. Gene Ther. 4, 432-441), versions of which transactivator are now also commercialized under the name "GeneSwitch" (Invitrogen Corporation, Carlsbad, CA), and rapamycin-regulated chimeric factor NFκB-FRB/FKBP-ZFHD1 (Rivera, V.M., Clackson, T., Natesan, S., Pollock, R., Amara, J.F., Keenan, T., Magari, S.R., Philips, T., Courage, N.L., Cerasoli, F. Jr., Holt, D.A., and Gilman, M. 1996. Nat. Med. 2, 1028-1032; Magari, S.R., Rivera, V.M., Iuliucci, J.D., Gilman, M., and Cerasoli, F.,jr. 1997. J. Clin. Invest. 100, 2865-2872). Other small molecule-regulatable transactivators may be used, provided that they can be employed to control the activity of a target gene without also causing widespread deregulation of genes of the host cell and provided further that the associated small molecule regulators have a sufficiently low toxicity for the host cell at their effective concentrations.

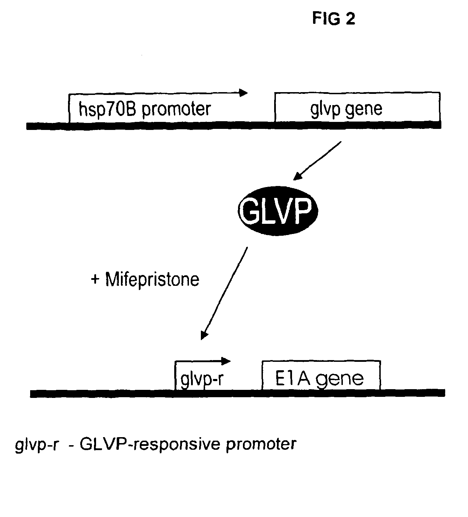

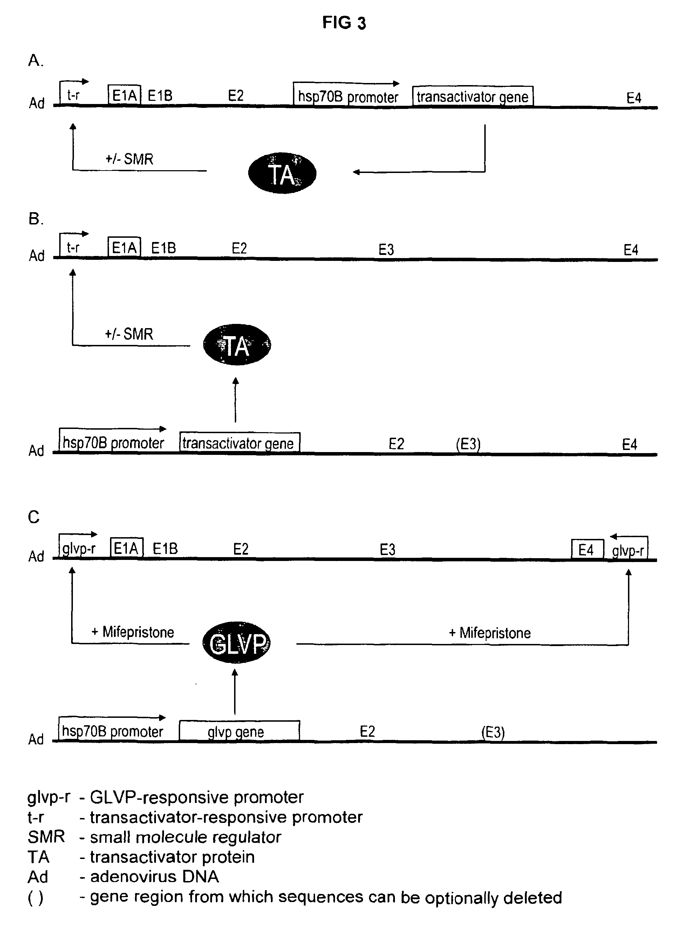

In the example in Figure 2, a gene for mifepristone-activatable, chimeric transactivator GLVP is functionally linked to strictly heat-regulated human hsp70B heat shock promoter. The target gene is a gene required for viral replication such as, in this example, the E1A gene of adenovirus. The target gene is functionally linked to a promoter that is responsive to the transactivator. In the present example, a suitable promoter will be a minimal promoter supplemented with binding sites for GAL4. A gene switch of the kind outlined in Figure 1 may be incorporated in a viral vector as shown in Figure 3 A. In this example, a transactivator-responsive promoter replaces the E1A promoter of adenovirus, and transactivator gene cassette replaces a non-essential gene segment of the virus, e.g., an E3 region in adenovirus. Alternatively, or if there is insufficient room in the deleted virus genome for inclusion of a chosen transactivator gene, the gene switch may be distributed between two viral vectors. In the example in Figure 3 B, a transactivator-responsive promoter replaces the E1A promoter of an otherwise wildtype adenovirus genome, and hsp70B-directed transactivator gene is inserted into the genome of a typical replication-deficient adenovirus lacking E1 and, possibly, E3 sequences. A specific example pair of conditionally replicating viruses of the invention is shown in Fig. 3 C. The first adenovirus is wildtype, except for E1A and E4 promoter regions that are replaced by transactivator-responsive promoters. The second virus is a replication-deficient virus that minimally lacks E1A and E4 functions. A hsp70B promoter - glvp gene cassette is inserted in the genome of the latter virus.

In order for viral replication to occur, cells will need to be co-infected with the recombinant virus pair. Gene switch-directed virus replication would be effected as follows: subsequent to infection of a cell with the recombinant virus combination of Figure 3 C, the cell would be exposed to an appropriate concentration of small molecule regulator mifepristone and subjected to a transient heat treatment to activate the hsp70B promoter. GLVP will be expressed and be activated by mifepristone. Active GLVP will then transactivate E1A and E4 gene expression from the first virus genome, resulting in replication of the virus pair. Cells in which the adenoviral replication occurred will lyse, virus particles will be liberated, and adjacent cells will be infected. Upon administration of a further transient heat treatment, replication in the secondarily infected cells will be triggered, and so forth.

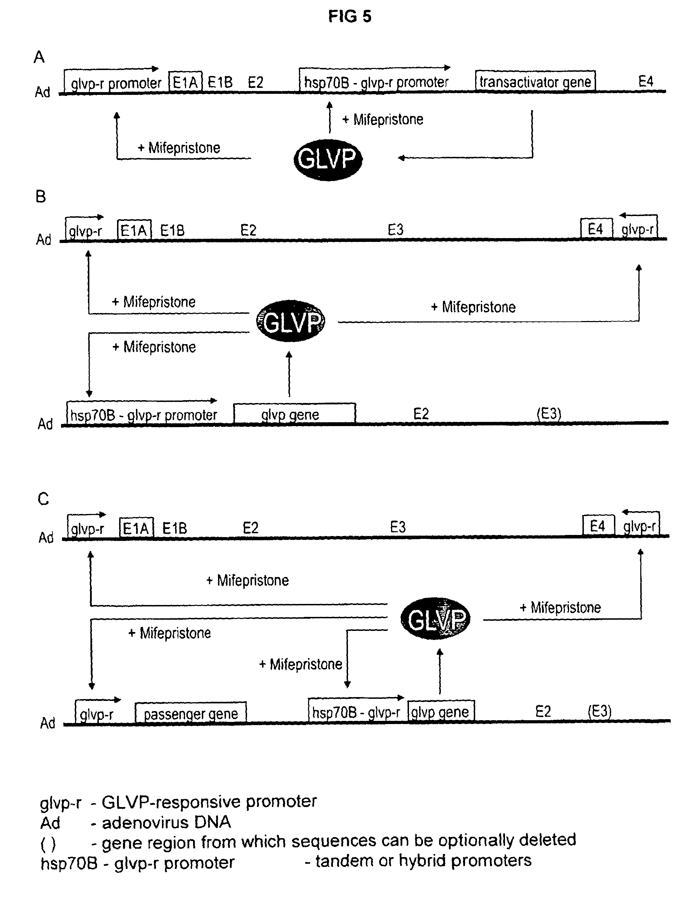

A virus or virus pair of the invention may also include a passenger gene. Whether the passenger gene or even a transactivator gene can be incorporated into a viral genome containing all genes and other sequences required for replication will depend on the type of virus, the maximal genome size for packaging, knowledge about non-essential viral genes that can be deleted, and the sizes of transactivator and passenger genes to be inserted. In the case of adenovirus, it may be necessary to insert the passenger gene and, possibly, even the transactivator gene into a partially deleted or completely gutted viral genome. As illustrated by the example case of Figure 5 C, the "virus of the invention" will be a pair of viruses, the first of which will contain a complete genome except for a substitution of E1A and E4 promoters with GAL4-binding site-containing glvp-r promoters that are responsive to GLVP transactivator. The second virus will be replication-deficient. Space provided by sizeable deletions of viral genes, typically E1 and E3 gene regions (and in this particular case also E4 sequences), will be taken by a passenger gene and a transactivator gene. The transactivator gene will be under the control of a functionally linked heat shock promoter, or the hybrid or tandem hsp70B-glvp-r promoter actually shown in the Figure (and discussed below). The passenger gene may be linked to a glvp-r promoter or another promoter, as the application may require.

It is noted that when a pair of viruses is used, the viruses are typically of the same species. The underlying reason is that typically at least one virus of the virus pair is dependent for replication on species-specific viral proteins expressed from genes carried by the other virus. It is further noted that it is not necessary that any one of the two viruses contain all genes required for replication. These genes may be distributed over both viruses. What is important is that, together, the two viral genomes encode all required viral functions.

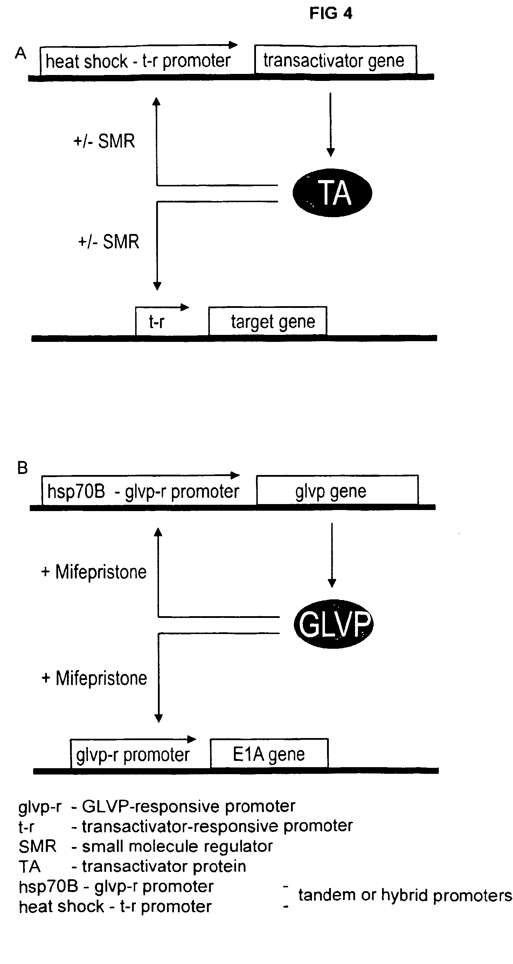

Most preferably, replication of viruses of the invention and, if desired, of passenger gene, is controlled by more complex switches described in U.S. patent No. 6,342,596 and in U.S. patent application Ser.No. 10/046420. Unlike the above-described switches, these switches contain a auto-activating element that allows them to remain active long after an activating heat treatment or exposure to other proteotoxic stress. A generic outline of the components and operation of such switches is shown in Figure 4 A. A switch of this kind also comprises two components. The first component is a gene for a small molecule-regulated transactivator, which gene is functionally linked to a DNA sequence that acts as a heat shock promoter as well as a transactivator-responsive promoter. This DNA sequence may contain a hybrid promoter comprising binding sites for both HSF1 and transactivator. Alternatively, it may contain two separate promoters, one of them a heat shock promoter and the other a transactivator-responsive promoter, provided that each of the promoters can direct expression from the linked transactivator gene. The second component is a target gene that is functionally linked to a transactivator-responsive promoter. When a cell containing the two components of such a switch is exposed to transient heat or other proteotoxic stress, HSF1 is activated and induces transcription from the transactivator gene. Depending on whether the particular transactivator used is activated or inhibited by a small molecule regulator, in the presence of absence of its small molecule regulator, newly made transactivator will activate the expression of the target gene as well as promote expression of additional transactivator protein. As a consequence, transactivator level is maintained after HSF1 has been inactivated subsequent to the transient stress and target gene expression continues.

Withdrawal or addition, respectively, of small molecule regulator will result in the inactivation of the switch. Figure 4 B shows an example a switch of this kind, in which the transactivator is mifepristone-activated transactivator GLVP and the target gene is the E1A gene of an adenovirus. In Figure 5, the incorporation of the latter example switch in adenoviruses of the invention is illustrated. Figure 5 A illustrates how the glvp gene and the linked hybrid or tandem promoter are introduced into the E3 region subsequent to deletion of sequences from this region. The E1A promoter is replaced by GAL4-binding site-containing promoter glvp-r that is responsive to GLVP. Whether such a recombinant can actually be made depends on the virus' packaging limit and on the lengths of the deleted and inserted sequences, respectively. In the example in Fig. 5 B, the switch is distributed over a pair of viruses. One of these viruses may contain a complete viral genome, except for replacement of the E1A and E4 promoters with glvp-r promoters. The other virus is a replication-deficient virus that may lack E1, E3 and E4 sequences, but contain the glvp gene and the linked hybrid or tandem promoter that is activatable by heat and active GLVP. As shown in the graph in Figure 5 C, a passenger gene may also be included in the defective genome of the latter virus. The passenger gene may be controlled by a transactivator-responsive promoter or another promoter.

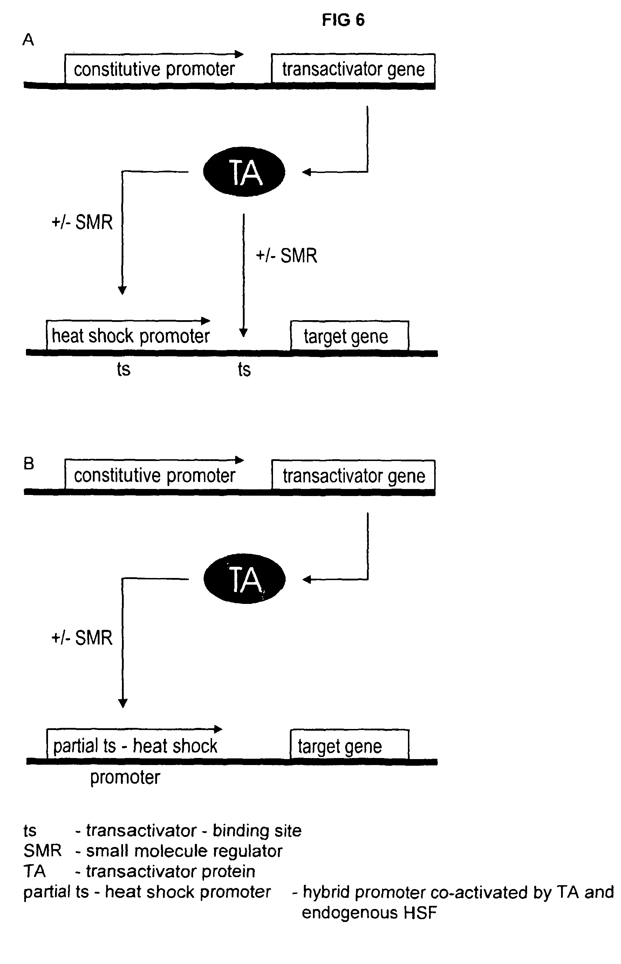

Alternatively, replication of a virus or virus pair of the invention and, if desired, expression of a passenger gene can be controlled by a type of gene switch (Figure 6 A) comprising a constitutively expressed gene for a transactivator (that is not transactivation-competent), whose DNA-binding activity is regulated by binding of a small molecule regulator, and a modified heat shock promoter, including 5'untranslated sequences, that contains one or more binding sites for the transactivator. The latter promoter is functionally linked to a viral or passenger gene to be regulated. Transactivator binding sites are so placed in the heat shock promoter (and/or RNA leader region) that binding of transactivator prevents heat activated transcription of the linked gene. Binding of transactivator to sites inserted near HSF1-binding sites may prevent HSF1 binding to the promoter; binding of transactivator to sites inserted in the 5' untranslated sequences may inhibit transcription elongation. The resulting gene switch is activated by transient heat in the presence or absence of small molecule regulator. An additional type of gene switch (Figure 6 B) that may be used to control replication of viruses of the invention and, if desired, expression of a passenger gene comprises a constitutively expressed gene for a transactivator, whose DNA-binding activity is activated or inhibited by binding of a small molecule regulator, and a modified heat shock promoter including binding sites for the transactivator. These binding sites, that may be low-affinity binding sites for the transactivator, or, possibly, half sites, are insufficient to allow activation of the promoter by the transactivator alone. The promoter is effectively co-activated by HSF1 induced by a transient heat or other proteotoxic stress and active transactivator. A modified heat shock promoter that requires co-activation may be obtained by deletion of all but one heat shock elements in a heat shock promoter, whose activation depends on the presence of multiple heat shock elements, and addition of one or more transactivator-binding sites.

Viruses particularly suitable for use in the present invention are those that were previously used as replicating or oncolytic viral vectors such as adenoviruses, herpesviruses, and retroviruses, including foamy viruses.

The biology and genetics of adenovirus are well understood (Russell, W.C. 2000. J. Gen. Virol. 81, 2573-2604; Hitt, M.M., Addison, C.L., and Graham, F.L. 1997. Advances in Pharmacology 40, 137-206). Persons skilled in the art know how to prepare and administer replication-competent or replication-deficient adenovirus vectors. Strategies used to control replication of adenovirus were recently reviewed (Post, D.E., Khuri, F.R., Simons, J.W., and Van Meir, E.G. 2003. Hum. Gene Ther. 14, 933-946). Typically, conditional replication of adenovirus was achieved by replacing the E1A, E1B or E4 viral promoter with a regulated or tissue-selective promoter of choice. In some cases, two of the latter viral promoters were replaced. For example, Hernandez-Alcoceba et al. (Hum. Gene Ther. 11, 2009-2024 (2000)) prepared a conditionally replicating type 5 adenovirus by replacement of the E1A and E4 promoters by truncated pS2 promoters containing binding sites for estrogen receptor. The recombinant virus replicated and lysed efficiently estrogen receptor-positive but not estrogen-receptor-negative cells. Yu et al. (Cancer Res. 59, 1498-1504 (1999)) constructed conditionally replicating adenovirus 764 by substituting the E1A and E1B promoters with a PSE (prostate-specific enhancer) promoter and a promoter fragment from a kallikrein 2 gene, respectively. Virus 764 efficiently replicated in and killed cells from a prostate tumor cell line but not cells from several other cell lines.

Herpesviruses are enveloped, double-stranded DNA viruses with large genomes (152 kbp for HSV-1). This large genome size had hampered genetic analyses and experiments in the past. This problem has been solved by the use of bacterial artificial chromosomes (BACs) that allow integration of an entire herpesvirus genome, enabling the use of efficient mutagenesis and recombination protocols to manipulate the viral genome (Wagner, M., Ruzsics, Z., and Koszinowski, U.H. 2002. Trends in Microbiol. 10,318-324). The products of at least two of the immediate early genes of herpesvirus, ICP4 and ICP27 are essential for viral replication (Burton, E.A., Bai, Q., Goins, W.F., and Glorioso, J. 2002. Curr. Opin. Biotechnol. 13, 424-428, and references cited therein). Manipulations that place one of these genes under the control of a regulated or tissue-selective promoter will produce a conditionally replicating herpesvirus. For example, Miyatake et al. (J. Virol. 71, 5124-5132 (1997)) made use of HSV-1 mutant d120 that lacked both copies of the a4 gene, the gene encoding ICP4, and inserted into this mutant an a4 gene copy that was functionally linked to a promoter containing an albumin enhancer and promoter element. The resulting recombinant herpesvirus replicated three log scales better in albumin-expressing cells than in other cells. Furthermore, the virus effectively inhibited growth of an albumin-expressing tumor xenograft. Herpesviruses can be made to preferentially replicate in mitotically active cells by deletion of genes for thymidine kinase, ICP6 or ICP34.5 (Harrington, K.J., Bateman, A.R., Melcher, A.A., Ahmed, A., and Vile, R.G. 2002. Clinical Oncology 14, 3-16, and references cited therein).

Retroviruses are single-stranded, diploid RNA viruses that have been intensively investigated and used as replication-defective vectors to transfer therapeutic genes (Vile, R.G., and Russell, S.J. 1995. Br. Med. Bull. 51, 12-30). Persons skilled in the art know how to manipulate retroviral genome and how to manufacture retroviral vectors. Preparation of retroviral vectors and their uses are described in many publications including European patent application EP 0178220; U.S. Pat. No. 4,405,712; Gilboa. 1986. Biotechniques 4, 504-512; Mann et al. 1983. Cell 33,153-159; Cone and Mulligan. 1984. Proc. Natl. Acad. Sci. USA 81, 6349-6353; Eglitis, M. A. et al. 1988. Biotechniques 6, 608-614; Miller, A. D. et al. 1989. Biotechniques 7, 981-990; Miller, A. D.1992. Curr. Top. Microbiol. Immunol. 158, 1-24; PCT application No. WO 92/07943 entitled "Retroviral Vectors Useful in Gene Therapy"; Hu, W.S., and Pathak, V.K. 2000. Pharmacol. Rev. 52, 493-511. Promoters driving the expression of viral genes reside in the U5 and U3 regions of provirus. The actual viral genome lacks the U5 region. Hence, it only contains the promoter of the U3 region. Recently, Logg et al. (J. Virol. 76, 12783-12791 (2002)) demonstrated that replacement of the promiscuous transcriptional control elements within the U3 region in murine leukemia virus with sequences from the promoter of the androgen-regulated probasin gene that is active in the prostate, or from a variant promoter derived from the probasin promoter, resulted in recombinant virus that preferentially replicated in prostate-derived cells. Nestler et al. (Gene Ther. 4, 1270-1277 (1997) reported the construction of a replication-competent foamy virus that lacked a large segment of the U3 region and most of the bet gene. Genes encoding prodrug-activating enzymes, i.e., herpes thymidine kinase, cytosine deaminase and polynucleoside phosphorylase were fused to the truncated bet gene. The latter "suicide" proteins were expressed as fusion proteins, which were processed to yield unfused proteins due to the presence of a self-cleaving sequence at the fusion point.

More generally, viruses suitable for use in the invention include retroviruses, and single-stranded and double-stranded DNA viruses that utilize the host transcriptional machinery and whose replication depends on proteins expressed post infection. The latter DNA viruses include the Hepadnaviridae, Parvoviridae, Papovaviridae, Circoviridae, Adenoviridae and Herpesviridae families.

Papovaviridae include at least nine human papilloma viruses and polyomavirus BK and JC.

Retroviridae include those of genera Lentivirus, Spornavirus and others such as HTLV-I, HTLV-II, HIV-I, HIV-II, bovine leukosis virus, feline sarcoma and leukemia viruses, avian reticuloendotheliosis, mouse mammary tumor virus, visna viruses, equine infectious anemia virus, FIV, bovine lentivirus, SUV, and foamy virus.

Hepadnaviridae include hepatitis B-like viruses, e.g. hepatitis B and other related viruses.

Parvoviridae includes those of genera parvovirus, dependovirus and densovirus, such as human and animal parvoviruses.

Adenoviridae include those of genera Mastadenovirus and Aviadenovirus, such human and animal mastadenovirus strains.

Herpesviridae include those of genera Simplexvirus, Varicellavirus, Betaherpesvirinae, Cytomegalovirus, lymphocryptovirus, Marek's disease-like viruses and Rhadinovirus. Examples are herpes simplex, varicella-zoster virus, human cytomegalovirus, Marek's disease virus, EB virus and others.

Circoviridae include the genus Circovirus. Example circoviruses are chicken anemia virus, psittacine beak and feather disease virus, and porcine circovirus.

Extensive descriptions of the biology, biochemistry and genetics of the above virus families and their known members are found in "Virology" (B.N. Fields, D.M. Knipe, and P.M. Howley, eds.), vol. 1 and 2, Lippincott-Raven Publishers, Philadelphia, PA. (1996).

Passenger genes may include cell-affecting genes such as genes that encode apoptopic inducers, genes that affect cell death, aging, division and DNA synthesis, mitochrondial genes, peroxisomal genes, immune response-related genes, ATP-binding proteins, cytoskeletal genes, all rescue genes, genes involved in cell damage and repair. A listing of potential yeast and mammalian genes which may be included in the viral vectors of the invention is provided below. Yeast Genes:
CELL RESCUE, DEFENSE, CELL DEATH AND AGING PRE3, PRE1, PUP2, RPN12, RPT1, MAG1, OGG1, SED1, ATH1, SPE2, GRE3, TPS2, TPS1, ATR1, ATX1, SK13, SK12, SK18, APN1, HPR5, ERG5, CCZ1, SRA1, SNF1, YCK1, YCK2, HRR25, CTA1, CTT1, WSC4, PAM1, TIR2, TIR1, HDF2, TFB4, RAD1, HAM1, LYS7, SOD1, KIN28, DIT2, ERG11, CYC7, CCP1, PHR1, DAK2, DAK1, ALR1, ALR2, HOR2, RAD17, DDC1, DDR2, ALK1, HEL1, SSL2, RAD5, SGS1, PIF1, RAD3, CDC9, REV7, NTG1, RAD18, RAD57, RAD55, XRS2, RAD30, MMS21, RAD51, RAD10, PS02, REV1, DIN7, RAD54, CDC2, PES4, POL2, REV3, RPB7, RPB4, SGE1, UBA1, UBC4, UBC5, RAD6, QR18, RNC1, NTG2, ERC1, RAD4, ETH1, FKB2, YHB1, FLR1, MEC3, ZWF1, GSH1, GRX1, TTR1, HYR1, GLR1, YCF1, FPS1, GPD1, RAS2, RAS1, CUPS, HSP26, HSP30, HSP12, HSP104, DDR48, HSC82, HSP82, MDJ1, MDJ2, HSP60, HSP78, ECM10, SSE1, SSA1, SSA3, SSA4, SSA2, SSE2, HSF1, HIG1, HDF1, HMS2, GRE1, DD11, RTA1, SIMI, LAG2, ZDS1, MET18, SNG1, NCA3, KTI12, UTH1, SUN4, SSU81, SSD1, TH14, KAR3, LIF1, SFA1, LAG1, LTV1, MDR1, SSK22, SSK2, HOL1, CIS3, HSP150, PIR3, MAC1, CUP1 A, CUP1 B, YDJ1, SSQ1, SSC1, IMP2, MPT5, ATX2, SN02, MLP1, NHX1, NCP1, NSR1, SNF4, RAD16, RAD7, RAD14, RAD23, ROD1, MGT1, OSM1, SIP18, SAT2, MNR2, MMS2, PNT1, CYP2, PAD1, PDRS, PDR3, PDR6, RTS1, PA13, HOR7, DUN1, IRE1, MKK2, MET22, PPZ2, PTC1, PTP2, MMS4, RAD52, PDR13 SLG1, GRR1 HIT1, RDH54 BRO1, PIR1 MSRA, RNR4 RNR3, HAL1, YGP1, CDC55, PPZ1, PKC1, HAL5, MKK1, HOG1, SLT2, BCKi, RAD53, SIR4, SIR3, SIR2, MGA1, FUN30, YR02, DNL4, RRD1, SAT4, RAD27, MSN2, ST11, PAU3, PAU2, PAU5, PAU1, PAU4, PAU6, (MLP1), RAD2, FZ_F1, SSU1, SOD2, CRS5, BCK2, ASM4, TIP1, TFB1, CCL1, SSL1, TFB3, TFB2, TSA1, TRX1, TRX2, ROX3, PDR1, GTS1, MCM1, SKN7, CAD 1, MSN4, YAP1, SLN1, SSK1, PBS2, UB14, RSP5, SVS1, ZRC1
CELL GROWTH, CELL DIVISION AND DNA SYNTHESIS GSC2, PLC1, PRE3, PRE2, PRE1, PUP2, RPN12, RPT6, RPT1, DIS3, RP SOA, AGA1, AGA2, ASG7, ACH1, ACT1, SAC6, ARP100, ABP1, PAN1, ARP 2, AREIARE2, SPE2, CYR1, SRV2, ADK2, GCS1, SOH1, TUB1, TUB3 SAG1, AKR1, YAR1, SK18,
ARG82, ABF1, STE6, BAR1, 8011, TUB2, RBI-2, BIG1, BI M1, BAT1, BEM1, BEM4, SBE2, BN14, BUD6, 8012, BUD9, BUD4, BUD8, RC K2, CMK1, CNA1, CMP2, CNB1, CCH1, CMD1, SRA1, YCK1, YCK2, HRR2 5, CKA2, CKA1, YCK3, EST2, TFS1, SCM4, GIC2, GIC1, CAK1, BUB2, B UB3, ESR1, RAD24, DBF20, PDS1, HPC2, NUD1, CDC47, CDC10, CDC13, C DC37, CDC1, CDC40, CDC4, CDC20, CDC6, CDC46, CDC3, KAR1, BB P1, CDC50, FUS1, KRE9, EGT2, ARP1, CHS1, CHS2, CHS3, CHS5, MS11, CAC2, R LF2, CHL4, SMC1, SMC2, CIN1, SNF7, CLC1, COF1, PAM1, LAS17, HDF2, SEC3, SNF2, SWI1, SNF5, SNF1 1, DOC1, APC2, APC5, TAP42, CDC53, KAR 9, CCE1, CLB6, CLB5, CLN3, PCL2, CLN1, PCL1, CLN2, CI-133, CI-131, CLB 4, CI-132, FAR1, CKS1, CDC28, PH085, KIN28, SSN3, CLG1, DIT2, SLA1, SLA2, SP020, DPP1, RAD17, CDC1, HEL1, DNA2, RAD5, SGS1, HCS I, PIF1, CDC9, MSH3, MSH6, MLH1, PMS1, MSH2, MSH1, POL4, REV 7, MRE11, RAD26, RAD9, RAD18, RAD57, RAD55, XRS2, MMS21, RAD51, RADIO, RAD 50, RFA3, RFA2, RFA1, RFC4, RFC5, RFC3, RFC2, RFC1, FOB1, TOP1, TO P2, TOP3, RAP1, RAD54, PR12, PR11, POL1, POI-12, CTF4, HUS2, CDC2, P ES4, POL2, DPB2, DPB3, MIP1, REV3, SSN8, GAL11, RGR1, SRB6, RP041, SEC59, DIP2, CDC14, MSG5, DYN1, UBC4, UBC9, CDC34, UBC5, UBC 1, UBC6, RAD6, QR18, ELC1, RNC1, CTS1, KEX2, APG1, SSP1, SUP35, EXM2, S PR1, EXG1, EXG2, DHS1, CAP1, CAP2, BRN1, GPR1, GIF1, MEC3, TU B4, CIS2, LTE1, SDC25, SRM1, CDC25, ROM2, BUDS, ROM1, SPT16, CDC43, G IP1, SIN4, SNF6, KRE6, GFA1, NGR1, WH12, RSR1, CIN4, RAS2, RAS1, GP A1, STE4, STE18, CDC42, MDG1, SEC4, TEM1, RH03, RH04, RI-102, RHO 1, CDC24, BEM2, BUD2, BEM3, LRG1, GPA2, SIS1, HSP82, HSF1, ABF2, HDF1, HDR1, RPD3, HSL7, HO, SBA1, HPR1, IDS2, NFI1, CSE2, MDM 1, MI-1 131, MIDI, SIMI, HIR3, SIS2, MAKI 1, LAS1, SPA2, WH14, ECM33, SET1, CTF19, CIN2, MCM16, SLK19, CYK2, CNM67, SST2, DPB11, DOS2, D FG16, AFR1, ZDS1, SR07, PEA2, FAR3, SMP2, WH13, CDC5, MET30, SAS2, SCC2, CIS 1, STN1, UTH1, PAC2, SSD1, SRP1, KRE5, KIP1, CIN8, SMY1, KIP2, KAR3, KIP3, CBF1, CBF2, SKP1, CEP3, CTF13, DBR1, LAG1, MIH1, BFR1, DIG2, DIG1, MFA1, MFA2, MF Alpha1, MF Alpha2, MID2, SSF1, MATALPHA2, MATA LPHA1, ALPHA1, ALPHA2, A2, A1, SAN1, PGD1, SPO11, MSH5, DMC 1, ISC10, MSH4, SP013, NDT80, REC104, HOP1, RED1, SP07, MUM2,ME15, S AE2, NAM8, REC107, REC102, REC114, MER1, RIM01, NDJ1, CDC54, CP R7, SYG1,MCM2, CIS3, HSP150, ACE2, CDC48, ASE1, YTM1, HSM3, YDJ1, ERV1, FUS3, JNM1, MCD1, MMC1, MSB1, MSB2, MPT5, ZDS2, MSN5, KEM1, MLC1, MY02, MY04, MY05, MY03, MYO1, DEC1, PMD1, M DS3, ASH1, UME1, UME6, NHP6A, RFT1, TRF5, NNF1, NDC1, BIK1, KAR2, KAR5, NUM1, CDC39, MAK16, NAP1, RAD16, RAD23, NBP35, ORC1, ORC6, ORC5, ORC4, ORC3, RRR1, SIC 1, BUD3, PWP2, STE3, STE2, OPY2, STE50, STE5, PEL1, TOR1, TOR2, PIK1, STT4, MSS4, SP014, POL32, IME4, SHP1, PDS5, FEN1, CSE1, FL08, Pry1, PHB2, PHB1, POI-30, AXL1, STE23, RAD28, CDC7, SMP3, MKK2, CDC15, ARD1, CHU, PPH3, PPH21, PPH22, PTC1, SE C9, PPS1, PTP3, YVH1, PTP2, PUS4, PCH2, PCH1, CBF5, SEF1, MMS4, SHR5, RAD59, RAD52, RHC18, RGP1, RVS167, RIM9, BNR11, BN11, SPT3, SOK2, KAR 4, DBF4, SDS22, MCM3, CTF18, SR04, SPH1, FUS2, MOB1, FL08, FIG1, FIG2, END3, DFG5, CTR9, TOM1, POP2, GRR1, SCP160, SUR1, MUM3, ZIP2 CDC45, RDH54, SHE3, SHE2, SHE4, GPI1, MIF2, ESP1, HOP2, DNA43, SMC3, PAC11, PAC10, RD11, RGA1, RNR1, RNR2, RNR4, RNR3, PRPS 1, RPL10, RPS1A, MTF1, SN12, CDC12, CDC11, SPR28, CDC55, GLC7, PKC1, GI N4, SPS1, RCK1, BUB1, IME2, YAK1, YPK2, RIM11, CLA4, MKK1, MEK1, I PL1, SGV1, SLT2, KSS1, BCK1, STE11, STE20, DBF2, HSL1, NRK1, SIT4, T PD3, ELM1, MCK1, RAD53, STE7, SWE1, MPS1, SAS3, HST1, SIR4, SIR3, SIR1, SIR2, CTH1, DOM34, HST4, RVS161, DNL4, IQG1, FUN16, HYM1, RT S2, MNN10, PRK1, MCM6, SAP155, SAP4, SAP190, SAP185, MUD13, M AD1, CIK1, NUF1, SPC97, SPC42, SPC98, CDC31, NUF2, MAD3, MAD2, DI T1, YSW1, SP012, SP016, MCD4, BDF1, SGA1, GSG1, SHC1, CDA1, CD A2, SMK1, SPS2, SPR6, SLZI, SPS4, SPR3, SPS100, SPS18, RAD27, SNZ 1, SUR4, ST11, SBE22, CSE4, BMH 1, SVL3, SCH9, (MLP1), SSF2, RAD2, CDH1, CDC27, CDC26, CDC23, CDC16, APC1, APC 11, APC4, APC9, SAP30, RSC6, RSC8, STH1, SFH1, SAS5, JSN1, BMH2, SMT4, BCK2, HOC1, ZIP1, UFE1, EST1, TEL1, ANC1, CCL1, DST1, TRX1, TRX2, TRF4, PAT1, SPT4, SP T6, CDC36, SW15, SW14, PHD1, SW16, GTS1, MCM1, IME1, SKN7, MAP1, SW13, SIN3, STE12, CIN5, SDS3, SP01, MOT2, RPG1, PRT1, CDC33, TPM1, TPM2, TWF1, TEC1, TTP1, STE13, PRP8, UB14, DSK2, RSP5, D OA4, UNG1, VPS45, VAN1, VRP1, DFG10, YHM2, GL'Q3, SFP1, STE24, RME1, SAE3, ME14, NHP6B, MOB2, EST3, RIM I
HEAT SHOCK PROTEINS CAT5, CPH1, CTT1, CYP2, DDR2, FPR2, HSC82, HSP104, HSP12, HSP150, HSP26, HSP30, HSP42, HSP60, HSP78, HSP82, KAR2, MDJ1, SIS1, S OD2, SSA1, SSA2, SSA3, SSA4, SSB1, SSB2, SSC1, SSE1, SSE2, ST11, TIP 1, TPS2, UB14, YDJ1 MITOCHONDRIAL AAC1, AAC3, AAT1, ABC1, ABF2, AC01, ACR1, ADH3, ADK2, AEP2, AFG3, ALD1, ALD2, ARG11, ARG2, ARG5,6, ARG7, ARG8, ARH1, AT M 1, ATP1, ATP10, ATP11, ATP12, ATP14, ATP15, ATP16, ATP2, ATP3, ATP4, A TP5, ATP6, ATP7, ATP8, ATP9, BAT1, BCS1, CBP1, CBP2, CBP3, CBP4, CB P6, CBR1, CBS1, CBS2, CCA1, CCE1, CCP1, CEM1, CIT1, CIT3, COB, CO Q1, COQ2, COQ3, COQ6, COR1, COT1, COX1, COX10, COX11, COX12, C 0X3, COX14, COX15, COX17, COX2, COX3, COX4, COX5A, COX5B, COX6, COX7, COX8, COX9, CPR3, CTP1, CYB2, CYC1, CYC2, CYC3, CYC7, CYT1, CYT2, DB156, DLD1, DTP, ENS2, ERV1, FLX1, FUM1, GCV1, GCV3, Gl-04, GPD2, GSD2, GUT2, HEM1, HEM15, HSP10, HSP60, HSP78, HTS1, IDH1, ID H2, IDP1, IFM1, ILV1, ILV2, ILV3, ILV5, ILV6, IMP1, IMP2, INH1, ISM1, KG D1, KGD2, LAT1, LEU4, LIP5, LPD1, LYS12, LYS4, MAE1, MAM33, MAS1, MAS2, MBA1, MCR1, MDH1, MDJ1, MDJ2, MDM10, MDM12, MEF1, MEF2, MET13, MGE1, MGM101, MIP1, MIR1, MIS1, MMM1, MMTi, M MT2, MOD5, MOL1, MRF1, MRP1, MRP13, MRP17, MRP2, MRP20, MRP21, MRP4, MRP49, MRP51, MRP8, MRPL10, MRPL 11, MRPL13, MRPL15, MRPL16, MRPL 17, MRPL 19, MRPL2, MRPL20, MRPL23, MRPL24, MRPL25, MRPL27, MRPL28, MRP L3, MRPL31, MRPL32, MRPL33, MRPL35, MRPL36, MRPL37, MRPL38, MR PI-39, MRPL4, MRPL40, MRPL44, MRPL49, MRPL6, MRPL7, MRPL8, M RPL9, MRPS28, MRPS5, MRPS9, MRS1, MRS11, MRS2, MRS3, MRS4, MRS5, MSD1, MSE1, MSF1, MSH1, MSK1, MSM1, MSP1, MSR1, MS S1, MSS116, MSS18, MSS51, MST1, MSU1, MSW1, MSY1, MTF1, M T01, NAM1, NAM2, NAM9, ND11, NHX1, NUC1, OM45, ORFA04514, OSM1, OXA1, PDA1, PDB1, PDX1, PEL1, PET111, PET112, PET117, PET122, PET123, PET127, PET130, PET191, PET309, PET494, PET54, PET56, PET9, PETCR46, PI-1131, PHB2, PIF1, PIM1, POR1, POR2, PPA2, PSD1, PUT1, PUT2, QCR10, QCR2, QCR6, QCR7, QCR8, QCR9, RCAi, RF2, RIM 1, RIM2, RIP1, RML2, RNA12, RPM2, RP 041, SC01, SCO2, SD1-11, SDH2, SD1-13, SDI-14, SECY, SHM1, SHY1, SLS1, SMF2, SOD2, SOM1, SSC1, SS.COPYRGT.1, STF1, STF2, SUN4, SUV3, TIM17, TIM22, TIM23 TIM44, TIM54, TOM20, TOM22, TOM37, TOM40, TOM6, TOM7, TOM 70, TOM 72, TRM1, TUF1, UNG1, VAR1, YAH1, YAL011W, YAT1, YBL013 W, YCR024C, YDR041W, YDR115W, YDR116C, YER073W, YFH1, YGLO68W, YGR257C, YHM1, YHR075C, YHR148W, YJL200C, YJR113C, YKLO55C, YKL120W, YKL134C, YKL192C, YLR168C, YMC1, YMC2, YML025C, YMR188C, YMR31, YNL081 C, YNL306W, YNR036C, YNR037C, YOR221C, YPL013C, ETF-BETA
PEROXISOMAL CAT2, CIT2, CTA1, DAL7, EHD1, EHD2, FAA2, FAT2, FOX2, ICU, IDP 3, MDH3, MLS1, PEX11, PEX12, PEX13, PEX14, PEX17, PEX2, PEX3, PE X4, PEX6, PEX7, PEXB, POT1, POX1, PXA1, PXA2, SPS19, YBR204C, YDR 449C, YHR180W DNA-ASSOCIATED A1, A2, ABF1, ABF2, ADA2, ADE12, ADR1, ALPHA1, ALPHA2, ANC1, APN1, ARGR1, ARGR2, ARGR3, ARR1, ASH1, AZFI, BAS 1, BDF1, BR F1, BUR6, CAC2, CAD1, CAF17, CATB, CBF1, CBF2, CCE1, CCR4, CDC13, CDC36, CDC39, CDC46, CDC47, CDC54, CDC6, CDC7, CDC73, CDC9, CEF1, CEP3, CHA4, CHD1, CHU, CHL4, CRZ1, CSE1, CSE2, CSE4, CTF13, CUP2, CUP9, DAL80, DAL81, DAL82, DAT1, DBF4, DMC1, DNA2, DNA43, DNL4, D OS2, DOT6, DP131 1, DPB2, DPB3, DST1, ECM22, ENS2, EST1, EZL1, FCP1, FHL1, FKH1, FKH2, FL08, FZF1, GAL11, GAL4, GAT1, GBP2, GCN4, GCNS, GCR1, GCR2, GLN3, GL03, GTS1, GZF3, HAC1, HAP1, HAP2, HAP3, HAP4, HCM1, HDA1, HDF1, HFM1, HHF1, HHF2, HH01, HHT1, HHT2, HM01, HMS1, HMS2, H0, HOP1, HPR1, HPRS, HSF1, HTA1, HTA2, HTA3, HTB1, HT 62, IFH1, IME1, IME4, IN02, IN04, IXR1, KAR4, LEU3, LYS14, LYS20, LYS21, M AC 1, MAGI, MAL13, MAL23, MAL33, MATALPHA1, MATALPHA2, MBP1, MCD1, MCM1, MCM2, MCM3, MCM6, MED6, MER2, MET18, MET28, MET30, MET31, MET32, MET4, MGA2, MGT1, MIF2, MIG1, MIG2, MIP1, MLH 1, MOL1, MOT1, MPT4, MRE11, MSH1, MSH2, MSH3, MSH4, MSHS, MS11, MSN1, MSN2, MSN4, MTF1, NBN1, NC132, NDJ1, NGG1, NHP2, NHP6 A, NHP6B, NOT3, NUC2, OAF1, OP11, ORC1, ORC2, ORC3, ORC4, ORCS, ORC6, PAF1, PCH1, PCH2, PDR1, PDR3, PGD1, PHD1, PH02, PH04, PHR1, PIF1, PIP2, PMS1, POB1, POL1, POD2, POL2, POL3, POL30, PO L4, POP2, PPR1, PRI1, PR12, PS02, PUT3, RAD1, RAD10, RAD14, RAD 16, RAD18, RAD2, RAD23, RAD26, RAD27, RAD3, RAD4, RADS, RAD50, RAD51, PAD52, RAD54 RAD55, RAD57, RAD6, RAD7, RAP1, RAT-1, RCS1, REB1, REC102, RE C104, REC114, RED1, REG1, RET1, REV3, RFA1, RFA2, RFA3, RFC1, RFC2, RFC3, RFC4, RFCS, RGM1, RGT1, RIF1, RIF2, RIM1, RIM101, RLF2, RLM1, R ME1, RMS1, ROX1, ROX3, RPA12, RPA135, RPA14, RPA190, RPA34, RPA43, RPA49, RP131 0, RPB11, RPB2, RP133, RP134, RPBS, RPB6, RPB7, RPBB, RPB9, RPC10, RPC19, RPC25, RPC31, RPC34, RPC40, RPC53, RPC82, RPD3, RP021, RP031, RP041, RRN10, RRN11, RRN3, RRNS, RRN6, RRN7, RRN9, RSC4, RSC 6, RSC8, RTG1, RTG3, SASS, SEF1, SET1, SFH1, SFLI, SGS1, SIG1, SIN 3, SIN4, SIP2, SIP4, SIR1, SIR2, SIR3, SIR4, SKN7, SK01, SMC1, SMC 2, SMP1, SNF2, SNFS, SNF6, SOK2, SPKi, SPOL, SPS18, SPT10, SPT 15, SPT16, SPT2, SPT21, SPT23, SPT3, SPT4, SPT5, SPT6, SPTB, SRI 32, SRB4, SRBS, S RB6, SRB7, SR138, SR139, SSL2, SSN3, SSN6, SSNB, SSU72, STB4, STBS, S TE12, STH1, SUA7, SWI1, SW13, SW14, SW16, SWP73, TAF19, TAF25, TBF1, TEA1, TEC1, TFA1, TFA2, TF131, TF132, TF133, TFB4, TFC1, TFC2, TF C3, TFC4, TFCS, TFG1, TFG2, TH12, TOA1, TOA2, TOP1, TOP2, TOP3, TRF4, TS P1, TUP1, TYE7, UGA3, UME6, UNG1, USV1, XRS2, YAL019W, YAP1, YA P3, YAPS, YBL054W, YBR026C, YBR150C, YBR239C, YCR106W, YDR026 C, YDR060W, YDR213W, YER045C, YER184C, YFL052W, YIL036W, YIL 130W, YJL103C, YJL206C, YKL005C, YKL222C, YKR064W, YLL054C, YLRO 87C, YLR266C, YNL206C, YOL089C, YOR172W, YOR380W, YOX1, YPL133C, YPR008W, YPR196W, YRR1, ZAP1, ZIP1, ZU01
IMMUNOSUPPRESSANT FEN1, SSH4, SHR3 CYCLINS CCU, CLB1, CLB2, CLB3, CL134, CLBS, CLB6, CLG1, CLN1, CLN2, CLN 3, CTK2, PCL1, PCL10, PCL2, PCLS, PCL6, PCL7, PCLB, PCL9, PH080, S SNB, YBR095C
ATP-BINDING CASSETTE PROTEINS ADP1, ATM 1, CAF16, GCN20, MDL1, MDL2, PER10, PDR11, PDR12, PDR15, PDRS, PXA1, PXA2, SNQ2, STE6, YBT1, YCF1, YDL223C, YD R091C, YEF3B, YER036C, YHL035C, YKR103W, YKR104W, YLL015W, YNR070W, YOR011W, YOR1, YPL226W
CYTOSKELETAL ABP1, ACF2, ACT1, AFR1, AIP1, AIP2, ARP3, AUT2, AUT7, BEM1, BI M1, BN11, BN14, BUD3, BUD6, CAP1, CAP2, CDC10, CDC11, CDC12, CDC 3, CIN1, CIN2, CIN4, CMD1, COF1, CRN1, END3, GIC1, GIC2, GIN4, J NM1, KAR9, KIP2, KIP3, LAS 17, MDM1, MHP1, MY01, MY02, MY03, MY04, MY05, PFY1, RVS161, RVS167, SAC6, SAC7, SEC 1, SHE3, SHM2, SLA1, SL A2, SMY1, SMY2, SPA2, SPH1, SPR28, SPR3, SRV2, TIP1, TPM1, TPM2, TUB1, TUB2, TUB3, VPS16, VRP1 APOPTOSIS ATP1, ATP14, ATP15, ATP16, ATP2, ATP3, ATP4, ATPS, ATP6, ATP7, ATP8, ATP9, CYC1, SH01, SSK2, SSK22, SW13, SXM1
CELL RESCUE ACC1, ALD6, BCK1, BEM 1, BEM2, BIM1, BMH1, BMH2, CAN1, CBF1, CDC1, CDC14, CDC15, CDC20, CDC25, CDC28, CDC33, CDC37, CDC 42, CDC43, CDC53, CDC6, CHC1, CIN8, CKA1, CKA2, CLA4, CLB1, CLB2, CLB3, CLB4, CLB5, CLN1, CLN2, CLN3, CMP2, CNA1, COF1, CTT1, DBF2, DBF20, DPM1, ERG25, GIC1, GIC2, GPA1, GRR1, HCA4, HIS4, HOC1, FSF1, KAR1, KES1, KRE6, KSS1, MBP1, NMT1, ORC2, ORC5, PDE2, PEP12, PEP7, PKC1, P LC1, PMR1, POL30, PRP18, RAM1, RAS1, RAS2, RBL2, RED1, RFC1, RH01, RH03, RH04, SAC1, SEC13, SEC14, SEC22, SEC4, SET1, SIS2, SKP1, SPC98, SRA1, SR04, SRP1, SSA1, SSA2, SSA4, SSN8, STE20, STN 1, STT4, SUJ 3, SWE1, SW14, SW16, TEL1, TOR1, TUB1, TUB4, VMA1, YCK1, YCK2, YPT1
CELL DAMAGE APN1, BUB1, CDC28, CDC45, CDC46, CDC47, CDC54, CDC7, CLB1, CLB2, CLB3, CLB5, DDC1, DDR2, DDR48, DIN7, DUN1, ECM32, HSM3, IMP2, MEC1, MEC3, MGT1, MOL1, MRE11, MUS81, NTG1, PDS1, PGD1, P HR1, POL2, POL3, POL30, POL4, PR11, PS02, RAD14, RAD16, RAD17, RAD18, RA D24, RAD30, RAD51, RAD52, RAD54, RAD55, RAD57, RAD7, RAD9, RDH54, REV3, RFA1, RFC5, RNR1, RNR2, RNR3, RNR4, RPH1, SIC1, SML1, SP K1, STN1, STS1, TEL1, TFA1, TFA2, TUP1, UBC7, UB14, XBP1, YBR098W, YFH1
OTHER RELEVANT MUTANTS AND GENES Y-1,9520b, C658-K7, JPD 4, JPM 9, Cy32, E354, JC488, PSY 142, 01-2, Y217, JC787-9A, ML1-21, Y500,86-9C, GL1, GT5-1A, HD565A, PZ1, 127-4D, Y229, JC302-26B, JC482, LB2211-2B, MH41-7B/P21, erg 81, SEY6211, GL4, K335, MK20, MK34, DE4-3A, DE4-3B, DE4-3C, MMY011, UH 1-GRGZ, 2150-2-3a, Y211, DP1/517,943,1117, C658, 1252, H79.20.3, LB1-3B, C658-K42, R29B, LB54-3A, XW520-9A, ade7, D225-5A, 309, SDH1, SDH2, SDH3, SDH4, TCM62, PDE1, PDE2

### Mammalian Genes:

11-beta hydroxysteroid dehydrogenase type II, 12-lipoxygenase, 17-beta hydroxysteroid dehydrogenase, 60S ribosomal protein L6,6-Omethylguanine -DNA methyltransferase, Activating transcription factor 2, Activating transcription factor 3, Activating transcription factor 4, Activin beta E, Activin receptor type 11, Acyl-CoA dehydrogenase, Acyl CoA Carrier Protein, Adenine nucleotide translocator 1, Alanine aminotransferase, Alcohol dehydrogenase 1, Alcohol dehydrogenase 2, Alcohol dehydrogenase 3, Alcohol dehydrogenase 4, Alcohol dehydrogenase 5, Aldehyde dehydrogenase 1, Aldehyde dehydrogenase 2, Aldehyde dehydrogenase 3, Alpha 1-antitrypsin, Alpha-1 acid glycoprotein, Alpha-1 antichymotrypsin, Alpha-catenin, Alphatubulin, Apolipoprotein A1, Apolipoprotein A11, Apolipoprotein Clil, Apolipoprotein E, Aryl hydrocarbon receptor, Aspartate aminotransferase, mitochondrial, Ataxia telangeictasia, ATP-dependent helicase 11 (70 kDa), ATP-dependent helicase 11 (Ku80), BAG-1, BAK, Bax (alpha), Bcl-2, Bcl-xL, Beta-actin, Bilirubin UDP-glucuronosyl-transferase isozyme 1, Bilirubin UDP-glucuronosyl-transferase isozyme 2, Biliverdin reductase, Branched chain acylCoA oxidase, BRCA I, BR-cadherin, C4bbinding protein, c-abl, Calcineurin B, Calnexin, Calprotectin, Calreticulin, canalicular multispecific organic anion transporter, Carbonic Anhydrase 111, Carnitine palmitoyl-CoA transferase, Caspase 1, Caspase 2 (Nedd2), Caspase 3 (CPP32beta), Caspase 5 (ICE rellll), Caspase 6 (Mch2-alpha), Caspase 7 (Mch3alpha), Caspase 8 (FLICE), Catalase, CatecholOmethyltransferase, CCAAT/enhancer-binding protein alpha, CCAAT/enhancer-binding protein epsilon, Cell division cycle protein 2, Cell division cycle protein 20, Cell division cycle protein 25, Cellular retinoic acid binding protein 1, Cellular retinoic acid binding protein 2, cerb; c-fos, Checkpoint kinase-1, Cholesterol esterase, c-H-ras, cjun, Clusterin, c-myc, Complement component C3, Connexin 30, Connexin32, Connexin-40, Corticosteroid binding globulin, Corticotropin releasing factor, C-reactive protein, Creatine kinase b, Cyclin D1, Cyclin dependent kinase 1, Cyclin dependent kinase 4, Cyclin dependent kinase inhibitor 1A, Cyclin E, Cyclin G, Cyclin-dependent kinase 4 inhibitor (P116), Cyclindependent kinase 4 inhibitor B (P16), Cyclin-dependent kinase inhibitor P27Kip1, Cyclooxygenase 2, Cystic fibrosis transmembrane conductance regulator, Cytochrome P450 11A1, Cytochrome P450 17A, Cytochrome P450 1A1, Cytochrome P450 1A2, Cytochrome P450 1 B1, Cytochrome P450 2A1, Cytochrome P450 2A3, Cytochrome P450 2A6, Cytochrome P450 2131, Cytochrome P450 21310, Cytochrome P450 2132, Cytochrome P450 2C11, Cytochrome P450 2C12, Cytochrome P450 2C19, Cytochrome P450 2C9, Cytochrome P450 2D6, Cytochrome P450 2E1, Cytochrome P450 2F2, Cytochrome P450 3A1, Cytochrome P450 3A4, Cytochrome P450 4A, Cytochrome P450 4A1, Damage specific DNA binding protein p48 subunit, Defender against cell death-1, Deleted in colorectal cancer, Deltalike protein, Dihydrofolate reductase, Disulfide isomerase related protein (ERp72), DNA binding protein inhibitor ID2, DNA dependent helicase, DNA dependent protein kinase, DNA ligase 1, DNA ligase IV, DNA mismatch repair protein (MLH1), DNA mismatch repair protein (PMS2), DNA mismatch repair/binding protein (MSH3), DNA polymerase alpha, DNA polymerase beta, DNA polymerase beta, DNA repair and recombination homologue (RAD 52), DNA repair helicase 11 ERCC-3, DNA repair protein (RAD 50), DNA repair protein (XRCC1), DNA repair protein XP-D, DNA replication factor C (36 kDa), DNA topoisomerase 1, DNA topoisomerase 11, Dopamine beta-hydroxylase, DRA, Dynein light chain 1, E2F, Early growth regulated protein 1, E-Cadherin, ECE-1 (endothelin converting enzyme), Endothelin-1, Enolase alpha, Enoyl CoA hydratase, Eotaxin, Epidermal growth factor, Epoxide hydrolase, ERA-B, ERCC 1 (excision repair protein), ERCC 3 (DNA repair helicase 11), ERCC 5 (excision repair protein), ERCC 6 (excision repair protein), ERK1, Erythropoietin, Erythropoietin receptor, ESelectin, Estrogen receptor, Farnesol receptor, Fas antigen, Fas associated death domain (FADD), Fas ligand, Fas/Apo1 receptor, Fatty acid synthase, Fatty acyl-CoA oxidase, Fatty acyl-CoA synthase, FEN-1 (endonuclease), Fibrinogen gamma chain, Fibronectin receptor, FIC1, Filagrin, Flavin containing monooxygenase 1, Flavin containing monooxygenase 3, FosB, Fra-1, Fucosyl transferase (alpha-1,2fucosyltransferase), Gadd153, Gadd45, Gamma-glutamyl hydrolase precursor, Gamma-glutamyl transpeptidase, GCLR, GCLS, Glucocorticoid receptor, Glucose-6-phosphate dehydrogenase, Glucose-regulated protein 170, Glucose-regulated protein 58, Glucose-regulated protein 78, Glucoseregulated protein 94, Glutamicoxaloacetic transaminase, Glutamine-pyruvic transaminase, Glutathione peroxidase, Glutathione reductase, Glutathione S-transferase alpha subunit, Glutathione S-transferase 4a, Glutathione synthetase, Glyceraldehyde 3-phosphate dehydrogenase, GOS24 (zinc finger transcriptional regulator), Granulocyte-macrophage colony-stimulating factor, Growth-arrested-specific protein 1, Growth-arrested-specific protein 3, GT mismatch binding protein, H-cadherin, Heat shock protein 12, Heat shock protein 47, Heat shock protein 70, Heat shock protein 70.1, Heat shock protein 90, Helicase-like transcription factor, Heme binding protein 23, Heme oxygenase-1, Hepatic lipase, Hepatocyte growth factor, Hepatocyte growth factor activator, Hepatocyte growth factor receptor, Hepatocyte nuclear factor 4, Histone 2A, Histone 28, HMG CoA reductase, Hydroxyacyl CoA dehydrogenase, Hydroxysteroid sulfotransferase a, Hypoxanthine-guanine phosphoribosyltransferase, ICE-rel 11 (Caspase 4), ICH-2 cysteine protease=CASPASE 4, IkB-a, Insulin-like growth factor binding protein 1, Insulin-like growth factor binding protein 2, Insulin-like growth factor binding protien 3, Insulin-like growth factor I, Insulin-like growth factor 11, Integrin alpha, Integrin alpha L, Integrin betas, Integrin beta2, Intercellular adhesion molecule-1, Intercellular adhesion molecule-2, Intercellular adhesion molecule-3, Interferon gamma, Interferon inducible protein 10, Interferon inducible protein 15, Interleukin-1 alpha, Interleukin-12, Interleukin-2, Interleukin-4, Interleukin-5, Interleukin-6, Involucrin, JNK1 stress activated protein kinase, K-cadherin, Ki67, Lactate Dehydrogenase 8, Lactoferrin, Lipopolysaccharide binding protein, Lipoprotein lipase precursor, Liver fatty acid binding protein, L-myc, Low density lipoprotein receptor, Luteinizing hormone, Lysyl oxidase, Macrophage inflammatory protein-1 alpha, Macrophage inflammatory protein-1 beta, Macrophage inflammatory protein-2 alpha, Macrophage inflammatory protein-2 beta, Macrophage inflammatory protein-3 alpha, Macrophage inflammatory protein-3 beta, Malic enzyme, MAP kinase kinase, Matrix metal loproteinase 1, Matrix metal loproteinase-2, MDM-2, MET proto-oncogene, Metallothionein 1, Metallothionein 2, Metallothionein 3, Metallothionein IA, Metallothionein IG, Metalregulatory transcription factor-1, Mitogen activated protein kinase (P38), Mitogen inducible gene (mig-2), MOAT-B (MRP/organic anion transporter), Monoamine oxidase A, Monoamine oxidase B, Multidrug resistance-associated protein, Multidrug resistant protein-1, Multidrug resistant protein-2, Multidrug resistant protein-3 =cMOAT2, MUTL homologue (MLH1), MutS Homologue (MSH2), Myeloid cell differentiation protein-1, Na/taurocholate cotransporting polypeptide, NADPH cytochrome P450 -oxidoreductase, NADPH cytochrome P450 reductase, NADPH quinone oxidoreductase-1 (DTDiaphorase), Natural killer cell-enhancing factor B, N-cadherin, NF-kappaB (p65), Nitric oxide synthase-1, inducible, Nucleoside diphosphate kinase beta isoform, 0-6-alkylguanine-DNAalkyltran- sferase, OBcadherin 1, OB-cadherin 2, Octamer binding protein 1, Octamer binding protein 2, Octamer binding protein 3, Oncostatin M, Organic anion transporter 1, Organic anion transporter 3, Organic anion transporter K1, Organic anion transporting polypeptide 1, Organic cation transporter 1, Organic cation transporter 2, Organic cation transporter 3, Organic cation transporter N1, Organic cation transporter N2, Ornithine decarboxylase, Osteopontin, Oxygen regulated protein 150, p53, PAPS synthetase, P-cadherin, PEGS (progression elevated gene 3), Peroxisomal 3-ketoacyl-CoA thiolase 1, Peroxisomal 3-ketoacylCoA thiolase 2, Peroxisomal acyl-CoA oxidase, Peroxisomal fatty acyl-CoA oxidase, Peroxisome assembly factor 1, Peroxisome assembly factor 2, Peroxisome biogenesis disorder protein-1, Peroxisome biogenesis disorder protein-11, Peroxisome biogenesis disorder protein-4, Peroxisome hydratase, Peroxisome proliferator activated receptor alpha, Peroxisome proliferator activated receptor gamma, Phenol sulfotransferase, Phosphoenolpyruvate carboxykinase, Phosphoglyceride kinase, Phospholipase A2, Plasminogen activator inhibitor 2, Platelet derived growth factor B, Platelet/endothelial cell adhesion molecule-1, Poly (ADP ribose) polymerase, Proliferating cell nuclear antigen gene, Prostaglandin H synthase, Protein kinase C beta1, Protein-tyrosine phosphatase, Putative protein tyrosine phosphatase, RAID, RAID 51 homologue, RANTES, Ref I, Replication factor C, 40-kDa subunit (Al), Replication protein A (70 kDa subunit), Retinoblastoma, Retinoblastoma related protein (P 107), Retinoid X receptor alpha, Retinoid X receptor beta, Retinoid X receptor gamma, Ribonucleotide reductase M1 subunit, Ribosomal protein L13A, Ribosomal protein S9, RNA-dependent helicase, ROAT1 (renal organic anion transporter), Serum amyloid A1, Serum amyloid A2alpha, Sister of p-glycoprotein, Sodium/bile acid cotransporter, Sonic hedgehog gene, SQM1, Superoxide Dismutase Cu/Zn, Superoxide dismutase Mn, T-cell cyclophilin, Tenascin, Thiopurine methyltransferase, Thioredoxin, Thrombospondin 2, Thymidine kinase, Thymidylate synthase, Thymosin beta-10, Tissue inhibitor of metalloproteinases-1, Tissue transglutaminase, Transcription factor IID, Transferrin, Transforming growth factor-beta 3, Tumor necrosis factor associated factor 2 (TRAF2), Tumor necrosis factor receptor 1, Tumor necrosis factor receptor 2, Tumor necrosis factor receptor-1 associated protein (TRADD), Tumor necrosis factor-alpha, Tumor necrosis factorbeta, Type I interstitial collagenase, Tyrosine aminotransferase, Tyrosine protein kinase receptor (UFO), Ubiquitin, Ubiquitin conjugating enzyme (Rad 6 homologue), Ubiquitin-homology domain protein PIC1, UDPglucuronosyltransferase 1, UDP-glucuronosyltransferase 1A6, UDPglucuronosyltransferase 2, UDP-glucuronosyltransferase 28, Uncoupling protein 1, Uncoupling protein 2, Uncoupling protein 3, Urate oxidase, UV excision repair protein RAD 23 (XP-C), Vascular cell adhesion molecule I (VCAM-1), Vascular endothelial growth factor, Vascular endothelial growth factor D, Very long-chain acyl-CoA dehydrogenase, Vimentin, Vitellogenin, Waf1, XRCC1 (DNA repair protein).

Passenger genes also include genes encoding prodrug-activating enzymes such as viral thymidine kinase, viral thymidine phosphorylase, cytosine deaminase, bacterial carboxypeptidase G2, cytochrome P450 proteins, carboxylesterase, deoxycytidine kinase, nitroreductase, purine nucleoside phosphorylase, horseradish peroxidase, and xantine guanine phosphoribosyl transferase. Also included are genes encoding "proteotoxins" such as diphtheria toxin and cytolethal distending toxin.

The present invention, thus generally described, will be understood more readily by reference to the following examples, which are provided by way of illustration and are not intended to be limiting of the present invention.

### EXAMPLES

### Construction of an adenovirus pair that conditionally replicates in infected cells exposed to small molecule regulator mifepristone and transient heat

Adenovirus type 5 is abbreviated Ad below. Generally known molecular biology and biochemistry methods are used. Molecular biology methods are described, e.g., in "Current protocols in molecular biology, Ausubel, F.M. et al., eds., volumes 1-4, John Wiley and Sons, Inc. ISBN 0-471-50338-X.

### Recombinant adenovirus 1 (rAd1)

Recombinant adenovirus I lacks nucleotides 28,130-30,820 encompassing E3. Nucleotide numbers relating to the adenovirus type 5 genome are as defined in GI: 33694637. Davison, A.J., Benko, M., and Harrach, B. 2003. J. Gen. Virol. 84, 2895-2908. Further, E1A promoter and E4 promoter sequences are replaced with a GAL4 site-containing minimal promoter.

A simplified system for generating recombinant adenovirus developed by He et al. (Proc. Natl. Acad. Sci. USA 95, 2509-2514 (1998)) is employed. A version of this system has bee made available commercially by Stratagene Corp. of La Jolla, California. A manual entitled "AdEasy^{™} Adenoviral Vector System" (revision no. 060002) is distributed by the Company to its customers and is also available at the Company's website www.stratagene.com.

Mutagenesis is carried out in transfer vector pShuttle (He et al. 1998. Proc. Natl. Acad. Sci. USA 95, 2509-2514) distributed by Statagene Corporation. The complete nucleotide sequence of this plasmid is identified in SEQ ID NO 4. According to Stratagene's manual entitled "AdEasy^{™} Adenoviral Vector System" (revision no. 060002), pShuttle contains the following adenovirus sequence elements: left inverted terminal repeat, encapsidation signal (Ad 1-331), "right arm homology region" (3,534-5790), "left arm homology region" (34,931-35,935) and right inverted terminal repeat. A site-directed mutagenesis procedure, the so-called QuikChange^{R} procedure of Stratagene Corp. that makes use of the polymerase chain reaction (PCR) is employed to delete E4 promoter sequences present in the left arm homology region of pShuttle (Ad 35,586-35,808) and replace them by an NheI restriction site. The QuikChange protocol is described, for example, in instruction manual "QuikChange XL site-directed mutagenesis kit" (revision no. 063003) published by Stratagene Corp. at their website www.stratagene.com and distributed to customers by mail. A related procedure is described briefly in Guettouche, T. (2002). Ph.D. Thesis, University of Miami, Miami, FL. The resulting construct is labeled "pShuttle-Nhe".

A GAL4-binding site-containing minimal promoter is inserted into the NheI site of pShuttle-Nhe. To achieve this, a DNA fragment encompassing nucleotides 7677 to 323 of plasmid pGene/V5-His (Invitrogen Corporation of Carlsbad, CA) is PCR-amplified using appropriate primers also containing an Nhel restriction site. After digestion of the PCR fragment with NheI, it is ligated to NheI-digested pShuttle-Nhe DNA. Recombinants are identified by restriction digestion and by nucleotide sequencing. Nucleotide sequencing permits the selection of a clone in which the inserted promoter sequence is correctly oriented relative to the E4 sequences in the left arm homology region of pShuttle-Nhe. This plasmid is named pShuttle-Nhe-GAL. The nucleotide sequence of pGene/V5-His is provided in SEQ ID NO 2. The plasmid is also described in instruction manual "GeneSwitch TM System, Version B, 000829/25-0313" of Invitrogen Corp. that is distributed by the Company to its customers and is also available at www.invitrogen.com.

Next, the right arm homology region of pShuttle-Nhe-GAL is replaced with Ad sequences also containing the beginning of the E1 region in addition to the homology region. pShuttle-Nhe-GAL is digested with BgIII and Pmel, filled in with DNA polymerase Klenow fragment and religated to produce pShuttle-Nhe-GAL-delE1. The latter plasmid lacks the right arm homology region. A fragment containing Ad 496-5780 is obtained by PCR amplification from plasmid pXC1 (Microbix Corporation, Toronto, ON). The nucleotide sequence of this plasmid is presented as SEQ ID NO 3. The latter PCR amplification is carried out using a forward primer (reading into the E1 gene) containing AscII and NotI restriction sites and a reverse primer containing a NotI restriction site. After digestion with NotI, the PCR fragment is ligated to Notl-digested pShuttle-Nhe-GAL-delE1 DNA to produce plasmid pShuttle-Nhe-GAL-E1. A clone is chosen in which the orientation of the inserted E1 sequence is the same as that of the truncated E1 sequence in the deleted right arm homology. In a subsequent subcloning step, a DNA fragment encompassing nucleotides 7677 to 323 of plasmid pGene/V5-His is PCR-amplified using appropriate primers also containing a AscII restriction site. After digestion of the PCR fragment with AscII, it is ligated to AscII-digested pShuttle-Nhe-GAL-E1 DNA. Recombinants are identified by restriction digestion and by nucleotide sequencing. Nucleotide sequencing permits the selection of a clone in which the inserted pGene/V5-His promoter sequence is correctly oriented relative to the E1 sequences. This clone is named pShuttle-Nhe-GAL-E1-GAL. Finally, the NotI site next to the inserted GAL4 site-containing promoter (inserted pGene/V5-His promoter sequence) is deleted by QuikChange mutagenesis. As a consequence, the resulting construct pShuttle-Nhe-GAL-E1-GAL-delNot contains a unique NotI site situated immediately downstream from the E I sequences. To prepare recombinant Ad, pShuttle-Nhe-GAL-E1-GAL-delNot DNA is linearized by NotI digestion and is co-electroporated with pAdEasy-1 DNA (He et al. 1998. Proc. Natl. Acad. Sci. USA 95, 2509-2514) into E.coli BJ5183 cells. BJ5183 cells (Stratagene catalog no. 200154) have the cellular components necessary to carry out homologous recombination between the introduced viral sequences. Detailed methods for the generation of recombinant Ad plasmids and the subsequent production of recombinant Ad viruses are discussed in He et al. (Proc. Natl. Acad. Sci. USA 95, 2509-2514 (1998)) and in Stratagene manual"AdEasy^{™} Adenoviral Vector System" (revision no. 060002). Briefly, recombinant Ad plasmids are characterized by restriction digestion. After preparation of a sufficient amount of DNA of a correct recombinant, the DNA is digested with PacI to separate plasmid sequences and inserted sequences comprising the Ad sequences. To produce recombinant Ad, the digested DNA is transfected into 293E4pIX cells. 293E4pIX cells are 293 cells containing an MMTV promoter-driven E4 transcription unit. In the presence of dexamethasone, these cells provide both E1 and E4 functions that are needed to amplify the desired recombinant virus that has conditionally active E1A and E4 genes. Using standard technology (briefly described in He et al.; see also Graham, F.L., and Prevec, L. 1991. Manipulation of adenovirus vectors. In: Methods in Molecular Biology, Gene Transfer and Expression Protocols, vol.7, ed. E.J. Murray, The Humana Press Inc., Clifton, NJ, pp. 109-127), virus plaques are isolated, and viral stocks are prepared. Viral stocks can be purified by CsCl gradient centrifugation. Note that pAdEasy-1 contains all Ad sequences except for nucleotides 1-3,533 and 28,130-30,820. Hence, the recombinant virus described here contains a deletion of a nonessential E3 region. By using an Ad plasmid that contains all E3 sequences, a recombinant virus that has a complete E3 region could be obtained.

### Recombinant adenovirus 2 (rAd2)

Recombinant adenovirus 2 is an E1/E3/E4-deleted Ad (lacking Ad 1-3,533, 28,130-30,820 and 35,586-35,808) that contains a transactivator that is related to transactivator GLVP described before. The transactivator employed in this example is a GAL4 DNA-binding domain-modified human progesterone receptor ligand-binding domain-human p65 activation domain chimera (GLP65) that can be retrieved from plasmid pSwitch (Invitrogen Corp.). The complete sequence of pSwitch is shown in SEQ ID NO 4. The transactivator is activated by estrogen antagonist mifepristone. In rAd2, the transactivator gene is functionally linked to an hsp70B-GAL4 tandem promoter. Optionally, rAd2 may also contain a passenger gene that is regulated by the hsp70B-GAL4 tandem promoter or another promoter.

hsp70B-GAL4 tandem promoter comprises a BamHI-HindIII fragment of about 460 bp length of the human hsp70B gene comprising the essential promoter sequences (about 350 bp), including the three functionally important heat shock element sequences, and an about 110 bp-long transcribed sequence (RNA leader region). Voellmy, R., et al. 1985. Proc. Natl. Acad. Sci. USA 82, 4949-4953. Schiller, P., et al. 1988. J. Mol. Biol. 203, 97-105. A unique restriction site was introduced close to the end of the transcribed sequence using the QuikChange procedure. Primers containing the same restriction site were used to PCR amplify the short intervening sequence (about 220 bp in length) present in plasmid phRL-CMV (Promega Corp., Madison, WI). After digestion, the PCR fragment was inserted into the added, unique restriction site in the hsp70B RNA leader region. An AscII site was then introduced into the intron sequence by the QuikChange procedure. A DNA fragment encompassing nucleotides 7677 to 323 of plasmid pGene/V5-His was PCR-amplified using appropriate primers also containing an AscII restriction site. After digestion of the PCR fragment with AscII, it was inserted into the intron AscII site. The resulting construct contained two promoters, an hsp70B promoter and a GAL4 site-containing promoter present in an intron that was inserted into the hsp70 RNA leader region. A gene placed downstream from this hsp70B-GAL4 tandem promoter can be transcribed from the hsp70B promoter that is activated by transient heat as well as from the GAL4 promoter that is activated by transactivator GLP65 in the presence of mifepristone. Note that a promoter that responds to heat and a GAL4 binding domain-containing transactivator does not need to be a tandem promoter. Hybrid promoters can also be constructed, e.g., by insertion of GAL4 binding sites into an hsp70B promoter. Note further that although a tandem or hybrid promoter provides the desired regulatory features that transcriptional activity of a linked gene is maintained subsequent to transient heat activation, in certain applications the use of such promoters is not absolutely required. In such applications, transactivator expression could be controlled, e.g., by the above-described 460-bp hsp70B promoter fragment.

To construct plasmid Shuttle-Nhe-hsp70B-GAL4-GLP65, a glp65-containing fragment encompassing nucleotides 500-2939 of pSwitch is PCR-amplified and inserted along with a fragment containing the Hsp70B-GAL4 tandem promoter into the multicloning site of pShuttle-Nhe. Restriction analysis and nucleotide sequencing are used to identify a clone, in which tandem promoter and glp65 sequences are joined in the proper relative orientation and in which transcription of glp65 proceeds clockwise, i.e., towards the right arm homology region. The pSwitch fragment contained in pShuttle-Nhe-hsp70B-GAL4-GLP65 includes the GLP65-coding sequence and a 3' untranslated region from a bovine growth hormone gene.

The rAD2 construct can also include a passenger gene. In this example IL 12-coding sequences and 3' untranslated sequences are PCR-amplified from an appropriate construct. This PCR fragment and a fragment containing the Hsp70B-GAL4 tandem promoter is inserted in the multicloning site next to the transactivator cassette. Nucleotide sequence analysis is used to verify that the Hsp70-GAL4 promoter is properly situated to control transcription from the il12 gene. It is noted that a single tandem promoter may be used to control glp65 and il12 gene activity. In this case the sequence of elements inserted in pShuttle-Nhe could be as follows: Hsp70B/GAL4 tandem promoter - il12 gene - internal ribosome binding site - glp65 gene - 3' untranslated sequences. Attention should be paid to the positioning of a passenger gene that is regulated by a constitutively active promoter. In such a case, a passenger gene cassette should be inserted downstream from the glp65 gene or in an orientation that results in an opposite transcriptional direction from that of the glp65 gene. It is noted further that any passenger gene and associated 3' untranslated sequences to be incorporated in rAd2 need to be screened for the presence of Pmel and PacI sites. If such sites are present, they need to be deleted. Deletion can typically be achieved conveniently by using the QuikChange mutagenesis procedure.

To prepare rAD2 (virus), rAD2 plasmid (pShuttle-Nhe-hsp70B-GAL4-GLP65 or a derivative plasmid) resulting from the above subcloning steps is linearized with PmeI, co-introduced with AdEasy-1 sequences in an appropriate E.coli strain to achieve homologous recombination of viral sequences, isolation of a correct recombinant plasmid, preparation of a sufficient amount of plasmid, digestion with PacI, transfection into 293 cells expressing E4, and isolation of rAd2 virus as described before.

Cells in a cell culture or cells in tissues infected with a mixture of rAd1 and rAd2 that are exposed to transient, directed heat in the presence of an appropriate concentration of mifepristone permit one round of replication of the virus pair (and expression of a passenger gene, if present, until cells are lysed). Viruses released from the initially infected cells infect surrounding cells. A second round of replication of the viruses can be triggered by a second transient, directed heat dose, and so forth.

Inevitably, in cells co-infected with rAd1 & 2, there is a low level of expression of GLP65 transactivator. This activity can be detected, e.g., by sensitive assays of passenger gene expression in the presence of mifepristone (without heat treatment). Any of the following measures reduces basal level expression of GLP65. Several of these measures can be combined for maximal effect. First, read-through transcriptional activity from tryptic, upstream promoters can be eliminated by inserting the tandem promoter - glp65 gene cassette in the opposite orientation, i.e., in the orientation in which the tandem promoter faces the right arm homology region of pShuttle-Nhe. Second, elimination of the 3' untranslated sequences adjacent to the glp65 gene also reduces basal expression. This can be achieved by co-introducing into the multicloning site of pShuttle-Nhe an Hsp70-GAL4 tandem promoter and a shortened PCR fragment containing nucleotides 500-2494 of pSwitch. Third, the sequence around the AUG at which translation of GLP65 begins (position 519-521 in pSwitch) can be modified to reduce translation efficiency. A reduction is achieved by changing the A in position -3 (relative to the A of the AUG start codon) to a T, and the G at position +4 to a T.

## Claims

1. A modified, conditionally replication-competent virus whose genome includes a gene switch activatable in an infected cell by exposure of the cell to heat and a small molecule regulator, the gene switch controlling the expression of a gene for a viral protein required for efficient replication of the modified virus.

2. A modified, conditionally replication-competent virus whose genome includes a gene switch that is activated in an infected cell by exposure of the cell to heat and is repressed by exposure of the cell to a small molecule regulator, the gene switch controlling the expression of a gene for a viral protein required for efficient replication of the modified virus.

3. The modified virus of claims 1 or 2, wherein the virus is a member of a family selected from the group consisting of Adenoviridae, Herpesviridae, and Retroviridae.

4. The modified virus of claims 1 or 2, wherein the virus is an adenovirus.

5. The modified virus of claim 4, wherein the gene switch controls the expression of at least one viral protein selected from the group consisting of E1A, E1B and E4.

6. A pair of modified viruses whose combined genomes contain all genetic information required for conditional replication of the virus pair; including a gene switch activatable in an infected cell by exposure of the cell to heat and a small molecule regulator, the gene switch controlling the expression of a gene for a viral protein required for efficient replication of the virus pair.

7. A pair of modified viruses whose combined genomes contain all genetic information required for conditional replication of the virus pair; including a gene switch that is activated in an infected cell by exposure of the cell to heat and is repressed by exposure of the cell to a small molecule regulator, the gene switch controlling the expression of a gene for a viral protein required for efficient replication of the virus pair.

8. The modified virus of claims 1 or 2, or the modified virus pair of claims 6 or 7, wherein the genome of the modified virus of claims 1 or 2, or the genome of at least one of the viruses of the virus pair of claims 6 or 7 further includes a therapeutic gene.

9. The modified virus of claims 1 or 2, or the modified virus pair of claims 6 or 7, wherein the genome of the modified virus of claims 1 or 2, or the genome of at least one of the viruses of the virus pair of claims 6 or 7 further includes an immune response-related gene.

10. The modified virus of claims 1 or 2, or the modified virus pair of claims 6 or 7, wherein the genome of the modified virus of claims 1 or 2 or the genome of at least one of the viruses of the virus pair of claims 6 or 7 further includes a gene selected from the group consisting of Granulocyte-macrophage colony-stimulating factor, Interferon gamma, Interleukin-1 alpha, Interleukin-12, Interleukin-2, Interleukin-4, Interleukin-5, Interleukin-6, Tumor necrosis factor-alpha and Tumor necrosis factor-beta.

11. The modified virus pair of claims 6 or 7, wherein the viruses are members of a family selected from the group consisting of Adenoviridae, Herpesviridae, and Retroviridae.

12. The modified virus pair of claims 6 or 7, wherein both viruses are adenoviruses.

13. The modified virus pair according to claim 12, wherein the gene switch controls the expression of at least one viral protein selected from the group consisting of E1A, E1B and E4.

14. The modified virus or virus pair according to any one of the above claims, for use as a medicament.

15. The modified virus or virus pair according to any one of claims 1 to 13, for use in the treatment of a tumor.

## Patentansprüche

1. Ein modifizierter, bedingt replizierfaehiger Virus dessen Genom einen Genschalter enthaelt, welcher in einer infizierten Zelle aktiviert wird, wenn die Zelle mit Hitze und einem niedrig-molekularen Regulator in Beruehrung gebracht wird, wobei der Genschalter die Expression eines Gens fuer ein virales Protein welches fuer die effiziente Replikation des modifizierten Virus benoetigt wird kontrolliert.

2. Ein modifizierter, bedingt replizierfaehiger Virus dessen Genom einen Genschalter enthaelt, welcher in einer infizierten Zelle aktiviert wird, wenn die Zelle mit Hitze in Beruehrung gebracht wird und welcher reprimiert wird durch Berehrung der Zelle mit einem niedrig-molekularen Regulator, wobei der Genschalter die Expression eines Gens fuer ein virales Protein welches fuer die effiziente Replikation des modifizierten Virus benoetigt wird kontrolliert.

3. Der modifizierte Virus nach Anspruch 1 oder 2, **dadurch gekennzeichnet, dass** der Virus ein Mitglied einer Familie ist, die ausgewaehlt ist aus der Gruppe bestehend aus Adenoviridae, Herpesviridae und Retroviridae.

4. Der modifizierte Virus nach Anspruch 1 oder 2, **dadurch gekennzeichnet, dass** der Virus ein Adenovirus ist.

5. Der modifizierte Virus nach Anspruch 4, **dadurch gekennzeichnet, dass** der Genschalter die Expession von mindestens einem viralen Protein kontrolliert, welches Protein ausgewaehlt ist aus der Gruppe bestehend aus E1A, E1B und E4.

6. Ein modifiziertes Viruspaar, dessen kombiniertes Genom alle genetische Information enthaelt die notwendig ist fuer die bedingte Replikation beider Viren, einschliesslich eines Genschalters, welcher in einer infizierten Zelle aktiviert wird, wenn die Zelle mit Hitze und einem niedrig-molekularen Regulator in Beruehrung gebracht wird, wobei der Genschalter die Expression eines Gens fuer ein virales Protein welches fuer die effiziente Replikation des modifizierten Viruspaars benoetigt wird kontrolliert.

7. Ein modifiziertes Viruspaar, dessen kombiniertes Genom alle genetische Information enthaelt die notwendig ist fuer die bedingte Replikation beider Viren, einschliesslich eines Genschalters, welcher in einer infizierten Zelle aktiviert wird, wenn die Zelle mit Hitze in Beruehrung gebracht wird und welcher reprimiert wird durch Berehrung der Zelle mit einem niedrig-molekularen Regulator, wobei der Genschalter die Expression eines Gens fuer ein virales Protein welches fuer die effiziente Replikation des modifizierten Viruspaars benoetigt wird kontrolliert.

8. Der modifizierte Virus nach Anspruch 1 oder 2, oder das modifizierte Viruspaar nach Anspruch 6 oder 7, **dadurch gekennzeichnet, dass** das Genom des modifizierten Virus nach Anspruch 1 oder 2, oder das Genom von wenigstens einem der Viren des Viruspaars nach Anspruch 6 oder 7 zusaetzlich ein therapeutisches Gen einschliesst.

9. Der modifizierte Virus nach Anspruch 1 oder 2, oder das modifizierte Viruspaar nach Anspruch 6 oder 7, **dadurch gekennzeichnet, dass** das Genom des modifizierten Virus nach Anspruch 1 oder 2, oder das Genom von wenigstens einem der Viren des Viruspaars nach Anspruch 6 oder 7 zusaetzlich ein der Immunantwort angehoeriges Gen einschliesst.

10. Der modifizierte Virus nach Anspruch 1 oder 2, oder das modifizierte Viruspaar nach Anspruch 6 oder 7, **dadurch gekennzeichnet, dass** das Genom des modifizierten Virus nach Anspruch 1 oder 2, oder das Genom von wenigstens einem der Viren des Viruspaars nach Anspruch 6 oder 7 zusaetzlich ein therapeutisches Gen einschliesst, das ausgewaehlt ist aus der Gruppe bestehend aus Granulozyten-Macrophagen Kolonie-stimulierendem Faktor, Interferon Gamma, Interleukin-1 Alpha, Interleukin-12, Interleukin-2, Interleukin-4, Interleukin-5, Interleukin-6, Tumor Nekrosis Faktor Alpha und Tumor Nekrosis Faktor Beta.

11. Das modifizierte Viruspaar nach Anspruch 6 oder 7, **dadurch gekennzeichnet, dass** die Viren Mitglieder einer Familie sind, die ausgewaehlt ist aus der Gruppe bestehend aus Adenoviridae, Herpesviridae und Retroviridae.

12. Das modifizierte Viruspaar nach Anspruch 6 oder 7, **dadurch gekennzeichnet, dass** beide Viren Adenoviren sind.

13. Das modifizierte Viruspaar nach Anspruch 12, **dadurch gekennzeichnet, dass** der Genschalter die Expession von mindestens einem viralen Protein kontrolliert, welches Protein ausgewaehlt ist aus der Gruppe bestehend aus E1A, E1B und E4.

14. Der modifizierte Virus oder das modifizierte Viruspaar nach irgendeinem der obengenannten Ansprueche, zur Benuetzung als Medikament.

15. Der modifizierte Virus oder das modifizierte Viruspaar nach einem der Ansprueche 1 bis 13, zur Benuetzung in der Behandlung eines Tumors.

## Revendications

1. Un virus modifié conditionnellement compétent à la réplication, dont le génome comprend un gène-interrupteur activable dans une cellule infectée par l'exposition de la cellule à la chaleur et une petite molécule régulatrice, le gène-interrupteur contrôlant l'expression d'un gène d'une protéine virale nécessaire à la réplication efficace du virus modifié.

2. Un virus modifié conditionnellement compétent à la réplication, dont le génome comprend un gène-interrupteur qui est activé dans une cellule infectée par l'exposition de la cellule à la chaleur et est réprimé par l'exposition de la cellule à une petite molécule régulatrice, le gène-interrupteur contrôlant l'expression d'un gène d'une protéine virale nécessaire à la réplication efficace du virus modifié.

3. Le virus modifié selon les revendications 1 ou 2, **caractérisée en ce que** le virus est un membre de la famille choisi parmi le group consistant en Adenoviridae, Herpesviridae, et Retroviridae.

4. Le virus modifié selon les revendications 1 ou 2, **caractérisée en ce que** le virus est un adénovirus.

5. Le virus modifié selon la revendication 4, **caractérisée en ce que** le gène-interrupteur contrôle l'expression d'au moins une protéine virale choisi parmi le group consistant en E1A, E1B et E4.

6. Une paire de virus modifiés dont les génomes combinés contiennent toute l'information génétique nécessaire à la réplication conditionnelle de la paire de virus ; comprenant un gène-interrupteur activable dans une cellule infectée par l'exposition de la cellule à la chaleur et une petite molécule régulatrice, le gène-interrupteur contrôlant l'expression d'un gène d'une protéine nécessaire à la réplication efficace de la paire de virus.

7. Une paire de virus modifiés dont les génomes combinés contiennent toute l'information génétique nécessaire à la réplication conditionnelle de la paire de virus ; comprenant un gène-interrupteur qui est activé dans une cellule infectée par l'exposition de la cellule à la chaleur et est réprimée par l'exposition de la cellule à une petite molécule régulatrice, le gène-interrupteur contrôlant l'expression d'un gène d'une protéine nécessaire à la réplication virale efficace de la paire de virus.

8. Le virus modifié selon les revendications 1 ou 2, ou la paire de virus modifiés selon les revendications 6 ou 7, **caractérisée en ce que** le génome du virus modifié selon les revendications 1 ou 2, ou le génome d'au moins un des virus de la paire de virus des revendications 6 ou 7 comprend en outre un gène thérapeutique.

9. Le virus modifié selon les revendications 1 ou 2, ou la paire de virus modifiés selon les revendications 6 ou 7, **caractérisée en ce que** le génome du virus modifié selon les revendications 1 ou 2, ou le génome d'au moins un des virus de la paire de virus des revendications 6 ou 7 comprend en outre un gène ayant un lien avec une réponse immunitaire.

10. Le virus modifié selon les revendications 1 ou 2, ou la paire de virus modifiés selon les revendications 6 ou 7, **caractérisée en ce que** le génome du virus modifié selon les revendications 1 ou 2, ou le génome d'au moins un des virus de la paire de virus des revendications 6 ou 7 comprend en outre un gène choisi parmi le group consistant en facteur stimulant colonie granulocyte macrophage, interféron-gamma, interleukine-1 alpha, interleukine-12, interleukine-2, interleukine-4, interleukine-5, interleukine-6, facteur de nécrose tumorale-alpha et facteur de nécrose tumorale-beta.

11. La paire de virus modifiés selon les revendications 6 ou 7, **caractérisée en ce que** les virus sont membres d'une famille choisi parmi le group consistant en Adenoviridae, Herpesviridae, et Retroviridae.

12. La paire de virus modifiés selon les revendications 6 ou 7, **caractérisée en ce que** les deux virus sont des adénovirus.

13. La paire de virus modifié selon la revendication 12, **caractérisée en ce que** le gène-interrupteur contrôle l'expression d'au moins une protéine virale choisi parmi le group consistant en E1A, E1B et E4.

14. Le virus modifié selon ou la paire de virus selon l'une les revendications précédentes, pour utilisation comme médicament.

15. Le virus modifié selon ou la paire de virus selon l'une les revendications 1 à 13, pour utilisation dans le traitement d'une tumeur.


### SEQUENCE LISTING

<110> HSF PHARMACEUTICALS S.A.
<120> VIRAL VECTORS CONTROLLED BY A GENE SWITCH
<130> Replicating virus
<140>
   <141>
<150> 10/731,961
   <151> 2003-12-10
<160> 4
<170> PatentIn version 3.3
<210> 1
   <211> 6621
   <212> DNA
   <213> Artificial
<220>
   <223> pShuttle transfer vector
<400> 1
   catcatcaat aatatacctt attttggatt gaagccaata tgataatgag ggggtggagt      60
   ttgtgacgtg gcgcggggcg tgggaacggg gcgggtgacg tagtagtgtg gcggaagtgt     120
   gatgttgcaa gtgtggcgga acacatgtaa gcgacggatg tggcaaaagt gacgtttttg     180
   gtgtgcgccg gtgtacacag gaagtgacaa ttttcgcgcg gttttaggcg gatgttgtag     240
   taaatttggg cgtaaccgag taagatttgg ccattttcgc gggaaaactg aataagagga     300
   agtgaaatct gaataatttt gtgttactca tagcgcgtaa tactggtacc gcggccgcct     360
   cgagtctaga gatatcgaat tcaagcttgt cgactcgaag atctgggcgt ggttaagggt     420
   gggaaagaat atataaggtg ggggtcttat gtagttttgt atctgttttg cagcagccgc     480
   cgccgccatg agcaccaact cgtttgatgg aagcattgtg agctcatatt tgacaacgcg     540
   catgccccca tgggccgggg tgcgtcagaa tgtgatgggc tccagcattg atggtcgccc     600
   cgtcctgccc gcaaactcta ctaccttgac ctacgagacc gtgtctggaa cgccgttgga     660
   gactgcagcc tccgccgccg cttcagccgc tgcagccacc gcccgcggga ttgtgactga     720
   ctttgctttc ctgagcccgc ttgcaagcag tgcagcttcc cgttcatccg cccgcgatga     780
   caagttgacg gctcttttgg cacaattgga ttctttgacc cgggaactta atgtcgtttc     840
   tcagcagctg ttggatctgc gccagcaggt ttctgccctg aaggcttcct cccctcccaa     900
   tgcggtttaa aacataaata aaaaaccaga ctctgtttgg atttggatca agcaagtgtc     960
   ttgctgtctt tatttagggg ttttgcgcgc gcggtaggcc cgggaccagc ggtctcggtc    1020
   gttgagggtc ctgtgtattt tttccaggac gtggtaaagg tgactctgga tgttcagata    1080
   catgggcata agcccgtctc tggggtggag gtagcaccac tgcagagctt catgctgcgg    1140
   ggtggtgttg tagatgatcc agtcgtagca ggagcgctgg gcgtggtgcc taaaaatgtc    1200
   tttcagtagc aagctgattg ccaggggcag gcccttggtg taagtgttta caaagcggtt    1260
   aagctgggat gggtgcatac gtggggatat gagatgcatc ttggactgta tttttaggtt 1320
   ggctatgttc ccagccatat ccctccgggg attcatgttg tgcagaacca ccagcacagt 1380
   gtatccggtg cacttgggaa atttgtcatg tagcttagaa ggaaatgcgt ggaagaactt 1440
   ggagacgccc ttgtgacctc caagattttc catgcattcg tccataatga tggcaatggg 1500
   cccacgggcg gcggcctggg cgaagatatt tctgggatca ctaacgtcat agttgtgttc 1560
   caggatgaga tcgtcatagg ccatttttac aaagcgcggg cggagggtgc cagactgcgg 1620
   tataatggtt ccatccggcc caggggcgta gttaccctca cagatttgca tttcccacgc 1680
   tttgagttca gatgggggga tcatgtctac ctgcggggcg atgaagaaaa cggtttccgg 1740
   ggtaggggag atcagctggg aagaaagcag gttcctgagc agctgcgact taccgcagcc 1800
   ggtgggcccg taaatcacac ctattaccgg gtgcaactgg tagttaagag agctgcagct 1860
   gccgtcatcc ctgagcaggg gggccacttc gttaagcatg tccctgactc gcatgttttc 1920
   cctgaccaaa tccgccagaa ggcgctcgcc gcccagcgat agcagttctt gcaaggaagc 1980
   aaagtttttc aacggtttga gaccgtccgc cgtaggcatg cttttgagcg tttgaccaag 2040
   cagttccagg cggtcccaca gctcggtcac ctgctctacg gcatctcgat ccagcatatc 2100
   tcctcgtttc gcgggttggg gcggctttcg ctgtacggca gtagtcggtg ctcgtccaga 2160
   cgggccaggg tcatgtcttt ccacgggcgc agggtcctcg tcagcgtagt ctgggtcacg 2220
   gtgaaggggt gcgctccggg ctgcgcgctg gccagggtgc gcttgaggct ggtcctgctg 2280
   gtgctgaagc gctgccggtc ttcgccctgc gcgtcggcca ggtagcattt gaccatggtg 2340
   tcatagtcca gcccctccgc ggcgtggccc ttggcgcgca gcttgccctt ggaggaggcg 2400
   ccgcacgagg ggcagtgcag acttttgagg gcgtagagct tgggcgcgag aaataccgat 2460
   tccggggagt aggcatccgc gccgcaggcc ccgcagacgg tctcgcattc cacgagccag 2520
   gtgagctctg gccgttcggg gtcaaaaacc aggtttcccc catgcttttt gatgcgtttc 2580
   ttacctctgg tttccatgag ccggtgtcca cgctcggtga cgaaaaggct gtccgtgtcc 2640
   ccgtatacag acttgagagg gagtttaaac gaattcaata gcttgttgca tgggcggcga 2700
   tataaaatgc aaggtgctgc tcaaaaaatc aggcaaagcc tcgcgcaaaa aagaaagcac 2760
   atcgtagtca tgctcatgca gataaaggca ggtaagctcc ggaaccacca cagaaaaaga 2820
   caccattttt ctctcaaaca tgtctgcggg tttctgcata aacacaaaat aaaataacaa 2880
   aaaaacattt aaacattaga agcctgtctt acaacaggaa aaacaaccct tataagcata 2940
   agacggacta cggccatgcc ggcgtgaccg taaaaaaact ggtcaccgtg attaaaaagc 3000
   accaccgaca gctcctcggt catgtccgga gtcataatgt aagactcggt aaacacatca 3060
   ggttgattca tcggtcagtg ctaaaaagcg accgaaatag cccgggggaa tacatacccg 3120
   caggcgtaga gacaacatta cagcccccat aggaggtata acaaaattaa taggagagaa 3180
   aaacacataa acacctgaaa aaccctcctg cctaggcaaa atagcaccct cccgctccag 3240
   aacaacatac agcgcttcac agcggcagcc taacagtcag ccttaccagt aaaaaagaaa 3300
   acctattaaa aaaacaccac tcgacacggc accagctcaa tcagtcacag tgtaaaaaag 3360
   ggccaagtgc agagcgagta tatataggac taaaaaatga cgtaacggtt aaagtccaca 3420
   aaaaacaccc agaaaaccgc acgcgaacct acgcccagaa acgaaagcca aaaaacccac 3480
   aacttcctca aatcgtcact tccgttttcc cacgttacgt aacttcccat tttaagaaaa 3540
   ctacaattcc caacacatac aagttactcc gccctaaaac ctacgtcacc cgccccgttc 3600
   ccacgccccg cgccacgtca caaactccac cccctcatta tcatattggc ttcaatccaa 3660
   aataaggtat attattgatg atgttaatta acatgcatgg atccatatgc ggtgtgaaat 3720
   accgcacaga tgcgtaagga gaaaataccg catcaggcgc tcttccgctt cctcgctcac 3780
   tgactcgctg cgctcggtcg ttcggctgcg gcgagcggta tcagctcact caaaggcggt 3840
   aatacggtta tccacagaat caggggataa cgcaggaaag aacatgtgag caaaaggcca 3900
   gcaaaaggcc aggaaccgta aaaaggccgc gttgctggcg tttttccata ggctccgccc 3960
   ccctgacgag catcacaaaa atcgacgctc aagtcagagg tggcgaaacc cgacaggact 4020
   ataaagatac caggcgtttc cccctggaag ctccctcgtg cgctctcctg ttccgaccct 4080
   gccgcttacc ggatacctgt ccgcctttct cccttcggga agcgtggcgc tttctcatag 4140
   ctcacgctgt aggtatctca gttcggtgta ggtcgttcgc tccaagctgg gctgtgtgca 4200
   cgaacccccc gttcagcccg accgctgcgc cttatccggt aactatcgtc ttgagtccaa 4260
   cccggtaaga cacgacttat cgccactggc agcagccact ggtaacagga ttagcagagc 4320
   gaggtatgta ggcggtgcta cagagttctt gaagtggtgg cctaactacg gctacactag 4380
   aaggacagta tttggtatct gcgctctgct gaagccagtt accttcggaa aaagagttgg 4440
   tagctcttga tccggcaaac aaaccaccgc tggtagcggt ggtttttttg tttgcaagca 4500
   gcagattacg cgcagaaaaa aaggatctca agaagatcct ttgatctttt ctacggggtc 4560
   tgacgctcag tggaacgaaa actcacgtta agggattttg gtcatgagat tatcaaaaag 4620
   gatcttcacc tagatccttt taaattaaaa atgaagtttt aaatcaatct aaagtatata 4680
   tgagtaaact tggtctgaca gttaccaatg cttaatcagt gaggcaccta tctcagcgat 4740
   ctgtctattt cgttcatcca tagttgcctg actccccgtc gtgtagataa ctacgatacg 4800
   ggagggctta ccatctggcc ccagtgctgc aatgataccg cgagacccac gctcaccggc 4860
   tccagattta tcagcaataa accagccagc cggaagggcc gagcgcagaa gtggtcctgc 4920
   aactttatcc gcctccatcc agtctattaa ttgttgccgg gaagctagag taagtagttc 4980
   gccagttaat agtttgcgca acgttgttgc cattgctgca gccatgagat tatcaaaaag 5040
   gatcttcacc tagatccttt tcacgtagaa agccagtccg cagaaacggt gctgaccccg 5100
   gatgaatgtc agctactggg ctatctggac aagggaaaac gcaagcgcaa agagaaagca 5160
   ggtagcttgc agtgggctta catggcgata gctagactgg gcggttttat ggacagcaag 5220
   cgaaccggaa ttgccagctg gggcgccctc tggtaaggtt gggaagccct gcaaagtaaa 5280
   ctggatggct ttcttgccgc caaggatctg atggcgcagg ggatcaagct ctgatcaaga 5340
   gacaggatga ggatcgtttc gcatgattga acaagatgga ttgcacgcag gttctccggc 5400
   cgcttgggtg gagaggctat tcggctatga ctgggcacaa cagacaatcg gctgctctga 5460
   tgccgccgtg ttccggctgt cagcgcaggg gcgcccggtt ctttttgtca agaccgacct 5520
   gtccggtgcc ctgaatgaac tgcaagacga ggcagcgcgg ctatcgtggc tggccacgac 5580
   gggcgttcct tgcgcagctg tgctcgacgt tgtcactgaa gcgggaaggg actggctgct 5640
   attgggcgaa gtgccggggc aggatctcct gtcatctcac cttgctcctg ccgagaaagt 5700
   atccatcatg gctgatgcaa tgcggcggct gcatacgctt gatccggcta cctgcccatt 5760
   cgaccaccaa gcgaaacatc gcatcgagcg agcacgtact cggatggaag ccggtcttgt 5820
   cgatcaggat gatctggacg aagagcatca ggggctcgcg ccagccgaac tgttcgccag 5880
   gctcaaggcg agcatgcccg acggcgagga tctcgtcgtg acccatggcg atgcctgctt 5940
   gccgaatatc atggtggaaa atggccgctt ttctggattc atcgactgtg gccggctggg 6000
   tgtggcggac cgctatcagg acatagcgtt ggctacccgt gatattgctg aagagcttgg 6060
   cggcgaatgg gctgaccgct tcctcgtgct ttacggtatc gccgctcccg attcgcagcg 6120
   catcgccttc tatcgccttc ttgacgagtt cttctgaatt ttgttaaaat ttttgttaaa 6180
   tcagctcatt ttttaaccaa taggccgaaa tcggcaacat cccttataaa tcaaaagaat 6240
   agaccgcgat agggttgagt gttgttccag tttggaacaa gagtccacta ttaaagaacg 6300
   tggactccaa cgtcaaaggg cgaaaaaccg tctatcaggg cgatggccca ctacgtgaac 6360
   catcacccaa atcaagtttt ttgcggtcga ggtgccgtaa agctctaaat cggaacccta 6420
   aagggagccc ccgatttaga gcttgacggg gaaagccggc gaacgtggcg agaaaggaag 6480
   ggaagaaagc gaaaggagcg ggcgctaggg cgctggcaag tgtagcggtc acgctgcgcg 6540
   taaccaccac acccgcgcgc ttaatgcgcc gctacagggc gcgtccattc gccattcagg 6600
   atcgaattaa ttcttaatta a 6621
<210> 2
   <211> 7697
   <212> DNA
   <213> Artificial
<220>
   <223> pGene/v5-His plasmid
<400> 2
   ccgagctctt acgcgggtcg aagcggagta ctgtcctccg agtggagtac tgtcctccga     60
   gcggagtact gtcctccgag tcgagggtcg aagcggagta ctgtcctccg agtggagtac    120
   tgtcctccga gcggagtact gtcctccgag tcgactctag agggtatata atggatctcg    180
   agatatcgga gctcgtttag tgaaccgtca gatcgcctgg agacgccatc cacgctgttt    240
   tgacctccat agaagacacc gggaccgatc cagcctccgc ggccgggaac ggtgcattgg    300
   aacgcgcatt ccccgtgtta attaacaggt aagtgtcttc ctcctgtttc cttcccctgc    360
   tattctgctc aaccttccta tcagaaactg cagtatctgt atttttgcta gcagtaatac    420
   taacggttct ttttttctct tcacaggcca ccaagcttgg taccgagctc ggatccacta 480
   gtccagtgtg gtggaattct gcagatcgaa acgatgatag atcccgtcgt tttacaacgt 540
   cgtgactggg aaaaccctgg cgttacccaa cttaatcgcc ttgcagcaca tccccctttc 600
   gccagctggc gtaatagcga agaggcccgc accgatcgcc cttcccaaca gttgcgcagc 660
   ctgaatggcg aatggcgctt tgcctggttt ccggcaccag aagcggtgcc ggaaagctgg 720
   ctggagtgcg atcttcctga ggccgatact gtcgtcgtcc cctcaaactg gcagatgcac 780
   ggttacgatg cgcccatcta caccaacgta acctatccca ttacggtcaa tccgccgttt 840
   gttcccacgg agaatccgac gggttgttac tcgctcacat ttaatgttga tgaaagctgg 900
   ctacaggaag gccagacgcg aattattttt gatggcgtta actcggcgtt tcatctgtgg 960
   tgcaacgggc gctgggtcgg ttacggccag gacagtcgtt tgccgtctga atttgacctg 1020
   agcgcatttt tacgcgccgg agaaaaccgc ctcgcggtga tggtgctgcg ttggagtgac 1080
   ggcagttatc tggaagatca ggatatgtgg cggatgagcg gcattttccg tgacgtctcg 1140
   ttgctgcata aaccgactac acaaatcagc gatttccatg ttgccactcg ctttaatgat 1200
   gatttcagcc gcgctgtact ggaggctgaa gttcagatgt gcggcgagtt gcgtgactac 1260
   ctacgggtaa cagtttcttt atggcagggt gaaacgcagg tcgccagcgg caccgcgcct 1320
   ttcggcggtg aaattatcga tgagcgtggt ggttatgccg atcgcgtcac actacgtctg 1380
   aacgtcgaaa acccgaaact gtggagcgcc gaaatcccga atctctatcg tgcggtggtt 1440
   gaactgcaca ccgccgacgg cacgctgatt gaagcagaag cctgcgatgt cggtttccgc 1500
   gaggtgcgga ttgaaaatgg tctgctgctg ctgaacggca agccgttgct gattcgaggc 1560
   gttaaccgtc acgagcatca tcctctgcat ggtcaggtca tggatgagca gacgatggtg 1620
   caggatatcc tgctgatgaa gcagaacaac tttaacgccg tgcgctgttc gcattatccg 1680
   aaccatccgc tgtggtacac gctgtgcgac cgctacggcc tgtatgtggt ggatgaagcc 1740
   aatattgaaa cccacggcat ggtgccaatg aatcgtctga ccgatgatcc gcgctggcta 1800
   ccggcgatga gcgaacgcgt aacgcgaatg gtgcagcgcg atcgtaatca cccgagtgtg 1860
   atcatctggt cgctggggaa tgaatcaggc cacggcgcta atcacgacgc gctgtatcgc 1920
   tggatcaaat ctgtcgatcc ttcccgcccg gtgcagtatg aaggcggcgg agccgacacc 1980
   acggccaccg atattatttg cccgatgtac gcgcgcgtgg atgaagacca gcccttcccg 2040
   gctgtgccga aatggtccat caaaaaatgg ctttcgctac ctggagagac gcgcccgctg 2100
   atcctttgcg aatacgccca cgcgatgggt aacagtcttg gcggtttcgc taaatactgg 2160
   caggcgtttc gtcagtatcc ccgtttacag ggcggcttcg tctgggactg ggtggatcag 2220
   tcgctgatta aatatgatga aaacggcaac ccgtggtcgg cttacggcgg tgattttggc 2280
   gatacgccga acgatcgcca gttctgtatg aacggtctgg tctttgccga ccgcacgccg 2340
   catccagcgc tgacggaagc aaaacaccag cagcagtttt tccagttccg tttatccggg 2400
   caaaccatcg aagtgaccag cgaatacctg ttccgtcata gcgataacga gctcctgcac 2460
   tggatggtgg cgctggatgg taagccgctg gcaagcggtg aagtgcctct ggatgtcgct 2520
   ccacaaggta aacagttgat tgaactgcct gaactaccgc agccggagag cgccgggcaa 2580
   ctctggctca cagtacgcgt agtgcaaccg aacgcgaccg catggtcaga agccgggcac 2640
   atcagcgcct ggcagcagtg gcgtctggcg gaaaacctca gtgtgacgct ccccgccgcg 2700
   tcccacgcca tcccgcatct gaccaccagc gaaatggatt tttgcatcga gctgggtaat 2760
   aagcgttggc aatttaaccg ccagtcaggc tttctttcac agatgtggat tggcgataaa 2820
   aaacaactgc tgacgccgct gcgcgatcag ttcacccgtg caccgctgga taacgacatt 2880
   ggcgtaagtg aagcgacccg cattgaccct aacgcctggg tcgaacgctg gaaggcggcg 2940
   ggccattacc aggccgaagc agcgttgttg cagtgcacgg cagatacact tgctgatgcg 3000
   gtgctgatta cgaccgctca cgcgtggcag catcagggga aaaccttatt tatcagccgg 3060
   aaaacctacc ggattgatgg tagtggtcaa atggcgatta ccgttgatgt tgaagtggcg 3120
   agcgatacac cgcatccggc gcggattggc ctgaactgcc agctggcgca ggtagcagag 3180
   cgggtaaact ggctcggatt agggccgcaa gaaaactatc ccgaccgcct tactgccgcc 3240
   tgttttgacc gctgggatct gccattgtca gacatgtata ccccgtacgt cttcccgagc 3300
   gaaaacggtc tgcgctgcgg gacgcgcgaa ttgaattatg gcccacacca gtggcgcggc 3360
   gacttccagt tcaacatcag ccgctacagt caacagcaac tgatggaaac cagccatcgc 3420
   catctgctgc acgcggaaga aggcacatgg ctgaatatcg acggtttcca tatggggatt 3480
   ggtggcgacg actcctggag cccgtcagta tcggcggaat tccagctgag cgccggtcgc 3540
   taccattacc agttggtctg gtgtcaaaaa gcggccgctc gaggtcaccc attcgaaggt 3600
   aagcctatcc ctaaccctct cctcggtctc gattctacgc gtaccggtca tcatcaccat 3660
   caccattgag tttaaacccg ctgatcagcc tcgactgtgc cttctagttg ccagccatct 3720
   gttgtttgcc cctcccccgt gccttccttg accctggaag gtgccactcc cactgtcctt 3780
   tcctaataaa atgaggaaat tgcatcgcat tgtctgagta ggtgtcattc tattctgggg 3840
   ggtggggtgg ggcaggacag caagggggag gattgggaag acaatagcag gcatgctggg 3900
   gatgcggtgg gctctatggc ttctgaggcg gaaagaacca gctggggctc tagggggtat 3960
   ccccacgcgc cctgtagcgg cgcattaagc gcggcgggtg tggtggttac gcgcagcgtg 4020
   accgctacac ttgccagcgc cctagcgccc gctcctttcg ctttcttccc ttcctttctc 4080
   gccacgttcg ccggctttcc ccgtcaagct ctaaatcggg ggctcccttt agggttccga 4140
   tttagtgctt tacggcacct cgaccccaaa aaacttgatt agggtgatgg ttcacgtagt 4200
   gggccatcgc cctgatagac ggtttttcgc cctttgacgt tggagtccac gttctttaat 4260
   agtggactct tgttccaaac tggaacaaca ctcaacccta tctcggtcta ttcttttgat 4320
   ttataaggga ttttgccgat ttcggcctat tggttaaaaa atgagctgat ttaacaaaaa 4380
   tttaacgcga attaattctg tggaatgtgt gtcagttagg gtgtggaaag tccccaggct 4440
   ccccagcagg cagaagtatg caaagcatgc atctcaatta gtcagcaacc aggtgtggaa 4500
   agtccccagg ctccccagca ggcagaagta tgcaaagcat gcatctcaat tagtcagcaa 4560
   ccatagtccc gcccctaact ccgcccatcc cgcccctaac tccgcccagt tccgcccatt 4620
   ctccgcccca tggctgacta atttttttta tttatgcaga ggccgaggcc gcctctgcct 4680
   ctgagctatt ccagaagtag tgaggaggct tttttggagg cctaggcttt tgcaaaaagc 4740
   tcccgggagc ttgtatatcc attttcggat ctgatcagca cgtgttgaca attaatcatc 4800
   ggcatagtat atcggcatag tataatacga caaggtgagg aactaaacca tggccaagtt 4860
   gaccagtgcc gttccggtgc tcaccgcgcg cgacgtcgcc ggagcggtcg agttctggac 4920
   cgaccggctc gggttctccc gggacttcgt ggaggacgac ttcgccggtg tggtccggga 4980
   cgacgtgacc ctgttcatca gcgcggtcca ggaccaggtg gtgccggaca acaccctggc 5040
   ctgggtgtgg gtgcgcggcc tggacgagct gtacgccgag tggtcggagg tcgtgtccac 5100
   gaacttccgg gacgcctccg ggccggccat gaccgagatc ggcgagcagc cgtgggggcg 5160
   ggagttcgcc ctgcgcgacc cggccggcaa ctgcgtgcac ttcgtggccg aggagcagga 5220
   ctgacacgtg ctacgagatt tcgattccac cgccgccttc tatgaaaggt tgggcttcgg 5280
   aatcgttttc cgggacgccg gctggatgat cctccagcgc ggggatctca tgctggagtt 5340
   cttcgcccac cccaacttgt ttattgcagc ttataatggt tacaaataaa gcaatagcat 5400
   cacaaatttc acaaataaag catttttttc actgcattct agttgtggtt tgtccaaact 5460
   catcaatgta tcttatcatg tctgtatacc gtcgacatct agctagagct tggcgtaatc 5520
   atggtcatag ctgtttcctg tgtgaaattg ttatccgctc acaattccac acaacatacg 5580
   agccggaagc ataaagtgta aagcctgggg tgcctaatga gtgagctaac tcacattaat 5640
   tgcgttgcgc tcactgcccg ctttccagtc gggaaacctg tcgtgccagc tgcattaatg 5700
   aatcggccaa cgcgcgggga gaggcggttt gcgtattggg cgctcttccg cttcctcgct 5760
   cactgactcg ctgcgctcgg tcgttcggct gcggcgagcg gtatcagctc actcaaaggc 5820
   ggtaatacgg ttatccacag aatcagggga taacgcagga aagaacatgt gagcaaaagg 5880
   ccagcaaaag gccaggaacc gtaaaaaggc cgcgttgctg gcgtttttcc ataggctccg 5940
   cccccctgac gagcatcaca aaaatcgacg ctcaagtcag aggtggcgaa acccgacagg 6000
   actataaaga taccaggcgt ttccccctgg aagctccctc gtgcgctctc ctgttccgac 6060
   cctgccgctt accggatacc tgtccgcctt tctcccttcg ggaagcgtgg cgctttctca 6120
   tagctcacgc tgtaggtatc tcagttcggt gtaggtcgtt cgctccaagc tgggctgtgt 6180
   gcacgaaccc cccgttcagc ccgaccgctg cgccttatcc ggtaactatc gtcttgagtc 6240
   caacccggta agacacgact tatcgccact ggcagcagcc actggtaaca ggattagcag 6300
   agcgaggtat gtaggcggtg ctacagagtt cttgaagtgg tggcctaact acggctacac 6360
   tagaagaaca gtatttggta tctgcgctct gctgaagcca gttaccttcg gaaaaagagt 6420
   tggtagctct tgatccggca aacaaaccac cgctggtagc ggtttttttg tttgcaagca 6480
   gcagattacg cgcagaaaaa aaggatctca agaagatcct ttgatctttt ctacggggtc 6540
   tgacgctcag tggaacgaaa actcacgtta agggattttg gtcatgagat tatcaaaaag    6600
   gatcttcacc tagatccttt taaattaaaa atgaagtttt aaatcaatct aaagtatata    6660
   tgagtaaact tggtctgaca gttaccaatg cttaatcagt gaggcaccta tctcagcgat    6720
   ctgtctattt cgttcatcca tagttgcctg actccccgtc gcgtagataa ctacgatacg    6780
   ggagggctta ccatctggcc ccagtgctgc aatgataccg cgagacccac gctcaccggc    6840
   tccagattta tcagcaataa accagccagc cggaagggcc gagcgcagaa gtggtcctgc    6900
   aactttatcc gcctccatcc agtctattaa ttgttgccgg gaagctagag taagtagttc    6960
   gccagttaat agtttgcgca acgttgttgc cattgctaca ggcatcgtgg tgtcacgctc    7020
   gtcgtttggt atggcttcat tcagctccgg ttcccaacga tcaaggcgag ttacatgatc    7080
   ccccatgttg tgcaaaaaag cggttagctc cttcggtcct ccgatcgttg tcagaagtaa    7140
   gttggccgca gtgttatcac tcatggttat ggcagcactg cataattctc ttactgtcat    7200
   gccatccgta agatgctttt ctgtgactgg tgagtactca accaagtcat tctgagaata    7260
   gtgtatgcgg cgaccgagtt gctcttgccc ggcgtcaata cgggataata ccgcgccaca    7320
   tagcagaact ttaaaagtgc tcatcattgg aaaacgttct tcggggcgaa aactctcaag    7380
   gatcttaccg ctgttgagat ccagttcgat gtaacccact cgtgcaccca actgatcttc    7440
   agcatctttt actttcacca gcgtttctgg gtgagcaaaa acaggaaggc aaaatgccgc    7500
   aaaaaaggga ataagggcga cacggaaatg ttgaatactc atactcttcc tttttcaata    7560
   ttattgaagc atttatcagg gttattgtct catgagcgga tacatatttg aatgtattta    7620
   gaaaaataaa caaatagggg ttccgcgcac atttccccga aaagtgccac ctgacgtcga    7680
   cggatcggga gatcgta                                                   7697
<210> 3
   <211> 9905
   <212> DNA
   <213> Artificial
<220>
   <223> p×C1 plasmid
<400> 3
   cccttccagc tctctgcccc ttttggattg aagccaatat gataatgagg gggtggagtt       60
   tgtgacgtgg cgcggggcgt gggaacgggg cgggtgacgt agtagtgtgg cggaagtgtg       120
   atgttgcaag tgtggcggaa cacatgtaag cgacggatgt ggcaaaagtg acgtttttgg       180
   tgtgcgccgg tgtacacagg aagtgacaat tttcgcgcgg ttttaggcgg atgttgtagt       240
   aaatttgggc gtaaccgagt aagatttggc cattttcgcg ggaaaactga ataagaggaa       300
   gtgaaatctg aataattttg tgttactcat agcgcgtaat atttgtctag ggccgcgggg       360
   actttgaccg tttacgtgga gactcgccca ggtgtttttc tcaggtgttt tccgcgttcc       420
   gggtcaaagt tggcgtttta ttattatagt cagctgacgt gtagtgtatt tatacccggt       480
   gagttcctca agaggccact cttgagtgcc agcgagtaga gttttctcct ccgagccgct       540
   ccgacaccgg gactgaaaat gagacatatt atctgccacg gaggtgttat taccgaagaa     600
   atggccgcca gtcttttgga ccagctgatc gaagaggtac tggctgataa tcttccacct     660
   cctagccatt ttgaaccacc tacccttcac gaactgtatg atttagacgt gacggccccc     720
   gaagatccca acgaggaggc ggtttcgcag atttttcccg actctgtaat gttggcggtg     780
   caggaaggga ttgacttact cacttttccg ccggcgcccg gttctccgga gccgcctcac     840
   ctttcccggc agcccgagca gccggagcag agagccttgg gtccggtttc tatgccaaac     900
   cttgtaccgg aggtgatcga tcttacctgc cacgaggctg gctttccacc cagtgacgac     960
   gaggatgaag agggtgagga gtttgtgtta gattatgtgg agcaccccgg gcacggttgc    1020
   aggtcttgtc attatcaccg gaggaatacg ggggacccag atattatgtg ttcgctttgc     1080
   tatatgagga cctgtggcat gtttgtctac agtaagtgaa aattatgggc agtgggtgat    1140
   agagtggtgg gtttggtgtg gtaatttttt ttttaatttt tacagttttg tggtttaaag    1200
   aattttgtat tgtgattttt ttaaaaggtc ctgtgtctga acctgagcct gagcccgagc    1260
   cagaaccgga gcctgcaaga cctacccgcc gtcctaaaat ggcgcctgct atcctgagac    1320
   gcccgacatc acctgtgtct agagaatgca atagtagtac ggatagctgt gactccggtc    1380
   cttctaacac acctcctgag atacacccgg tggtcccgct gtgccccatt aaaccagttg    1440
   ccgtgagagt tggtgggcgt cgccaggctg tggaatgtat cgaggacttg cttaacgagc    1500
   ctgggcaacc tttggacttg agctgtaaac gccccaggcc ataaggtgta aacctgtgat    1560
   tgcgtgtgtg gttaacgcct ttgtttgctg aatgagttga tgtaagttta ataaagggtg    1620
   agataatgtt taacttgcat ggcgtgttaa atggggcggg gcttaaaggg tatataatgc    1680
   gccgtgggct aatcttggtt acatctgacc tcatggaggc ttgggagtgt ttggaagatt    1740
   tttctgctgt gcgtaacttg ctggaacaga gctctaacag tacctcttgg ttttggaggt    1800
   ttctgtgggg ctcatcccag gcaaagttag tctgcagaat taaggaggat tacaagtggg    1860
   aatttgaaga gcttttgaaa tcctgtggtg agctgtttga ttctttgaat ctgggtcacc    1920
   aggcgctttt ccaagagaag gtcatcaaga ctttggattt ttccacaccg gggcgcgctg     1980
   cggctgctgt tgcttttttg agttttataa aggataaatg gagcgaagaa acccatctga    2040
   gcggggggta cctgctggat tttctggcca tgcatctgtg gagagcggtt gtgagacaca   2100
   agaatcgcct gctactgttg tcttccgtcc gcccggcgat aataccgacg gaggagcagc    2160
   agcagcagca ggaggaagcc aggcggcggc ggcaggagca gagcccatgg aacccgagag    2220
   ccggcctgga ccctcgggaa tgaatgttgt acaggtggct gaactgtatc cagaactgag    2280
   acgcattttg acaattacag aggatgggca ggggctaaag ggggtaaaga gggagcgggg     2340
   ggcttgtgag gctacagagg aggctaggaa tctagctttt agcttaatga ccagacaccg    2400
   tcctgagtgt attacttttc aacagatcaa ggataattgc gctaatgagc ttgatctgct    2460
   ggcgcagaag tattccatag agcagctgac cacttactgg ctgcagccag ggatgatttt    2520
   tgaggaggct attagggtat atgcaaaggt ggcacttagg ccagattgca agtacaagat    2580
   cagcaaactt gtaaatatca ggaattgttg ctacatttct gggaacgggg ccgaggtgga 2640
   gatagatacg gaggataggg tggcctttag atgtagcatg ataaatatgt ggccgggggt 2700
   gcttggcatg gacggggtgg ttattatgaa tgtaaggttt actggcccca attttagcgg 2760
   tacggttttc ctggccaata ccaaccttat cctacacggt gtaagcttct atgggtttaa 2820
   caatacctgt gtggaagcct ggaccgatgt aagggttcgg ggctgtgcct tttactgctg 2880
   ctggaagggg gtggtgtgtc gccccaaaag cagggcttca attaagaaat gcctctttga 2940
   aaggtgtacc ttgggtatcc tgtctgaggg taactccagg gtgcgccaca atgtggcctc 3000
   cgactgtggt tgcttcatgc tagtgaaaag cgtggctgtg attaagcata acatggtatg 3060
   tggcaactgc gaggacaggg cctctcagat gctgacctgc tcggacggca actgtcacct 3120
   gctgaagacc attcacgtag ccagccactc tcgcaaggcc tggccagtgt ttgagcataa 3180
   catactgacc cgctgttcct tgcatttggg taacaggagg ggggtgttcc taccttacca 3240
   atgcaatttg agtcacacta agatattgct tgagcccgag agcatgtcca aggtgaacct 3300
   gaacggggtg tttgacatga ccatgaagat ctggaaggtg ctgaggtacg atgagacccg 3360
   caccaggtgc agaccctgcg agtgtggcgg taaacatatt aggaaccagc ctgtgatgct 3420
   ggatgtgacc gaggagctga ggcccgatca cttggtgctg gcctgcaccc gcgctgagtt 3480
   tggctctagc gatgaagata cagattgagg tactgaaatg tgtgggcgtg gcttaagggt 3540
   gggaaagaat atataaggtg ggggtcttat gtagttttgt atctgttttg cagcagccgc 3600
   cgccgccatg agcaccaact cgtttgatgg aagcattgtg agctcatatt tgacaacgcg 3660
   catgccccca tgggccgggg tgcgtcagaa tgtgatgggc tccagcattg atggtcgccc 3720
   cgtcctgccc gcaaactcta ctaccttgac ctacgagacc gtgtctggaa cgccgttgga 3780
   gactgcagcc tccgccgccg cttcagccgc tgcagccacc gcccgcggga ttgtgactga 3840
   ctttgctttc ctgagcccgc ttgcaagcag tgcagcttcc cgttcatccg cccgcgatga 3900
   caagttgacg gctcttttgg cacaattgga ttctttgacc cgggaactta atgtcgtttc 3960
   tcagcagctg ttggatctgc gccagcaggt ttctgccctg aaggcttcct cccctcccaa 4020
   tgcggtttaa aacataaata aaaaaccaga ctctgtttgg atttggatca agcaagtgtc 4080
   ttgctgtctt tatttagggg ttttgcgcgc gcggtaggcc cgggaccagc ggtctcggtc 4140
   gttgagggtc ctgtgtattt tttccaggac gtggtaaagg tgactctgga tgttcagata 4200
   catgggcata agcccgtctc tggggtggag gtagcaccac tgcagagctt catgctgcgg 4260
   ggtggtgttg tagatgatcc agtcgtagca ggagcgctgg gcgtggtgcc taaaaatgtc 4320
   tttcagtagc aagctgattg ccaggggcag gcccttggtg taagtgttta caaagcggtt 4380
   aagctgggat gggtgcatac gtggggatat gagatgcatc ttggactgta tttttaggtt 4440
   ggctatgttc ccagccatat ccctccgggg attcatgttg tgcagaacca ccagcacagt 4500
   gtatccggtg cacttgggaa atttgtcatg tagcttagaa ggaaatgcgt ggaagaactt 4560
   ggagacgccc ttgtgacctc caagattttc catgcattcg tccataatga tggcaatggg 4620
   cccacgggcg gcggcctggg cgaagatatt tctgggatca ctaacgtcat agttgtgttc 4680
   caggatgaga tcgtcatagg ccatttttac aaagcgcggg cggagggtgc cagactgcgg 4740
   tataatggtt ccatccggcc caggggcgta gttaccctca cagatttgca tttcccacgc 4800
   tttgagttca gatgggggga tcatgtctac ctgcggggcg atgaagaaaa cggtttccgg 4860
   ggtaggggag atcagctggg aagaaagcag gttcctgagc agctgcgact taccgcagcc 4920
   ggtgggcccg taaatcacac ctattaccgg gtgcaactgg tagttaagag agctgcagct 4980
   gccgtcatcc ctgagcaggg gggccacttc gttaagcatg tccctgactc gcatgttttc 5040
   cctgaccaaa tccgccagaa ggcgctcgcc gcccagcgat agcagttctt gcaaggaagc 5100
   aaagtttttc aacggtttga gaccgtccgc cgtaggcatg cttttgagcg tttgaccaag 5160
   cagttccagg cggtcccaca gctcggtcac ctgctctacg gcatctcgat ccagcatatc 5220
   tcctcgtttc gcgggttggg gcggctttcg ctgtacggca gtagtcggtg ctcgtccaga 5280
   cgggccaggg tcatgtcttt ccacgggcgc agggtcctcg tcagcgtagt ctgggtcacg 5340
   gtgaaggggt gcgctccggg ctgcgcgctg gccagggtgc gcttgaggct ggtcctgctg 5400
   gtgctgaagc gctgccggtc ttcgccctgc gcgtcggcca ggtagcattt gaccatggtg 5460
   tcatagtcca gcccctccgc ggcgtggccc ttggcgcgca gcttgccctt ggaggaggcg 5520
   ccgcacgagg ggcagtgcag acttttgagg gcgtagagct tgggcgcgag aaataccgat 5580
   tccggggagt aggcatccgc gccgcaggcc ccgcagacgg tctcgcattc cacgagccag 5640
   gtgagctctg gccgttcggg gtcaaaaacc aggtttcccc catgcttttt gatgcgtttc 5700
   ttacctctgg tttccatgag ccggtgtcca cgctcggtga cgaaaaggct gtccgtgtcc 5760
   ccgtatacag acttgagagg cctgtcctcg gcctgtcctc gaccgatgcc cttgagagcc 5820
   ttcaacccag tcagctcctt ccggtgggcg cggggcatga ctatcgtcgc cgcacttatg 5880
   actgtcttct ttatcatgca actcgtagga caggtgccgg cagcgctctg ggtcattttc 5940
   ggcgaggacc gctttcgctg gagcgcgacg atgatcggcc tgtcgcttgc ggtattcgga 6000
   atcttgcacg ccctcgctca agccttcgtc actggtcccg ccaccaaacg tttcggcgag 6060
   aagcaggcca ttatcgccgg catggcggcc gacgcgctgg gctacgtctt gctggcgttc 6120
   gcgacgcgag gctggatggc cttccccatt atgattcttc tcgcttccgg cggcatcggg 6180
   atgcccgcgt tgcaggccat gctgtccagg caggtagatg acgaccatca gggacagctt 6240
   caaggatcgc tcgcggctct taccagccta acttcgatca ctggaccgct gatcgtcacg 6300
   gcgatttatg ccgcctcggc gagcacatgg aacgggttgg catggattgt aggcgccgcc 6360
   ctataccttg tctgcctccc cgcgttgcgt cgcggtgcat ggagccgggc cacctcgacc 6420
   tgaatggaag ccggcggcac ctcgctaacg gattcaccac tccaagaatt ggagccaatc 6480
   aattcttgcg gagaactgtg aatgcgcaaa ccaacccttg gcagaacata tccatcgcgt 6540
   ccgccatctc cagcagccgc acgcggcgca tctcgggcag cgttgggtcc tggccacggg 6600
   tgcgcatgat cgtgctcctg tcgttgagga cccggctagg ctggcggggt tgccttactg 6660
   gttagcagaa tgaatcaccg atacgcgagc gaacgtgaag cgactgctgc tgcaaaacgt   6720
   ctgcgacctg agcaacaaca tgaatggtct tcggtttccg tgtttcgtaa agtctggaaa   6780
   cgcggaagtc agcgccctgc accattatgt tccggatctg catcgcagga tgctgctggc   6840
   taccctgtgg aacacctaca tctgtattaa cgaagcgctg gcattgaccc tgagtgattt   6900
   ttctctggtc ccgccgcatc cataccgcca gttgtttacc ctcacaacgt tccagtaacc   6960
   gggcatgttc atcatcagta acccgtatcg tgagcatcct ctctcgtttc atcggtatca   7020
   ttacccccat gaacagaaat cccccttaca cggaggcatc agtgaccaaa caggaaaaaa   7080
   ccgcccttaa catggcccgc tttatcagaa gccagacatt aacgcttctg gagaaactca   7140
   acgagctgga cgcggatgaa caggcagaca tctgtgaatc gcttcacgac cacgctgatg   7200
   agctttaccg cagctgcctc gcgcgtttcg gtgatgacgg tgaaaacctc tgacacatgc   7260
   agctcccgga gacggtcaca gcttgtctgt aagcggatgc cgggagcaga caagcccgtc   7320
   agggcgcgtc agcgggtgtt ggcgggtgtc ggggcgcagc catgacccag tcacgtagcg   7380
   atagcggagt gtatactggc ttaactatgc ggcatcagag cagattgtac tgagagtgca   7440
   ccatatgcgg tgtgaaatac cgcacagatg cgtaaggaga aaataccgca tcaggcgctc   7500
   ttccgcttcc tcgctcactg actcgctgcg ctcggtcgtt cggctgcggc gagcggtatc   7560
   agctcactca aaggcggtaa tacggttatc cacagaatca ggggataacg caggaaagaa   7620
   catgtgagca aaaggccagc aaaaggccag gaaccgtaaa aaggccgcgt tgctggcgtt   7680
   tttccatagg ctccgccccc ctgacgagca tcacaaaaat cgacgctcaa gtcagaggtg   7740
   gcgaaacccg acaggactat aaagatacca ggcgtttccc cctggaagct ccctcgtgcg   7800
   ctctcctgtt ccgaccctgc cgcttaccgg atacctgtcc gcctttctcc cttcgggaag   7860
   cgtggcgctt tctcatagct cacgctgtag gtatctcagt tcggtgtagg tcgttcgctc   7920
   caagctgggc tgtgtgcacg aaccccccgt tcagcccgac cgctgcgcct tatccggtaa   7980
   ctatcgtctt gagtccaacc cggtaagaca cgacttatcg ccactggcag cagccactgg   8040
   taacaggatt agcagagcga ggtatgtagg cggtgctaca gagttcttga agtggtggcc   8100
   taactacggc tacactagaa ggacagtatt tggtatctgc gctctgctga agccagttac   8160
   cttcggaaaa agagttggta gctcttgatc cggcaaacaa accaccgctg gtagcggtgg   8220
   tttttttgtt tgcaagcagc agattacgcg cagaaaaaaa ggatctcaag aagatccttt   8280
   gatcttttct acggggtctg acgctcagtg gaacgaaaac tcacgttaag ggattttggt   8340
   catgagatta tcaaaaagga tcttcaccta gatcctttta aattaaaaat gaagttttaa   8400
   atcaatctaa agtatatatg agtaaacttg gtctgacagt taccaatgct taatcagtga   8460
   ggcacctatc tcagcgatct gtctatttcg ttcatccata gttgcctgac tccccgtcgt   8520
   gtagataact acgatacggg agggcttacc atctggcccc agtgctgcaa tgataccgcg   8580
   agacccacgc tcaccggctc cagatttatc agcaataaac cagccagccg gaagggccga   8640
   gcgcagaagt ggtcctgcaa ctttatccgc ctccatccag tctattaatt gttgccggga   8700
   agctagagta agtagttcgc cagttaatag tttgcgcaac gttgttgcca ttgctgcagg 8760
   catcgtggtg tcacgctcgt cgtttggtat ggcttcattc agctccggtt cccaacgatc 8820
   aaggcgagtt acatgatccc ccatgttgtg caaaaaagcg gttagctcct tcggtcctcc 8880
   gatcgttgtc agaagtaagt tggccgcagt gttatcactc atggttatgg cagcactgca 8940
   taattctctt actgtcatgc catccgtaag atgcttttct gtgactggtg agtactcaac 9000
   caagtcattc tgagaatagt gtatgcggcg accgagttgc tcttgcccgg cgtcaacacg 9060
   ggataatacc gcgccacata gcagaacttt aaaagtgctc atcattggaa aacgttcttc 9120
   ggggcgaaaa ctctcaagga tcttaccgct gttgagatcc agttcgatgt aacccactcg 9180
   tgcacccaac tgatcttcag catcttttac tttcaccagc gtttctgggt gagcaaaaac 9240
   aggaaggcaa aatgccgcaa aaaagggaat aagggcgaca cggaaatgtt gaatactcat 9300
   actcttcctt tttcaatatt attgaagcat ttatcagggt tattgtctca tgagcggata 9360
   catatttgaa tgtatttaga aaaataaaca aataggggtt ccgcgcacat ttccccgaaa 9420
   agtgccacct gacgtctaag aaaccattat tatcatgaca ttaacctata aaaataggcg 9480
   tatcacgagg ccctttcgtc ttcaagaatt ctcatgtttg acagcttatc atcgataagc 9540
   tttaatgcgg tagtttatca cagttaaatt gctaacgcag tcaggcaccg tgtatgaaat 9600
   ctaacaatgc gctcatcgtc atcctcggca ccgtcaccct ggatgctgta ggcataggct 9660
   tggttatgcc ggtactgccg ggcctcttgc gggatatcgt ccattccgac agcatcgcca 9720
   gtcactatgg cgtgctgcta gcgctatatg cgttgatgca atttctatgc gcacccgttc 9780
   tcggagcact gtccgaccgc tttggccgcc gcccagtcct gctcgcttcg ctacttggag 9840
   ccactatcga ctacgcgatc atggcgacca cacccgtcct gtggatccgg gcccccattt 9900
   cccct 9905
<210> 4
   <211> 7323
   <212> DNA
   <213> Artificial
<220>
   <223> pSwitch plasmid
<400> 4
   gacggatcgg gagatcattc gagcttgcat gcctgcaggt cgaagcggag tactgtcctc 60
   cgagtttaaa agcggagtac tgtcctccga ggatatcagc ggagtactgt cctccgagtc 120
   gcgaagcgga gtactgtcct ccgagatcga tgtcgacccc gcccagcgtc ttgtcattgg 180
   cgaattcgaa cacgcagatg cagtcggggc ggcgcggtcc gaggtccact tcgcatatta 240
   aggtgacgcg tgtggcctcg aatcgcctgg agacgccatc cacgctgttt tgacctccat 300
   agaagacacc gggaccgatc cagcctccgc ggccgggaac ggtgcattgg aacgcggatt 360
   ccccgtgtta attaacaggt aagtgtcttc ctcctgtttc cttcccctgc tattctgctc 420
   aaccttccta tcagaaactg cagtatctgt atttttgcta gcagtaatac taacggttct 480
   ttttttctct tcacaggcca ccaagctacc ggtccaccat ggactcccag cagccagatc    540
   tgaagctact gtcttctatc gaacaagcat gcgatatttg ccgacttaaa aagctcaagt    600
   gctccaaaga aaaaccgaag tgcgccaagt gtctgaagaa caactgggag tgtcgctact    660
   ctcccaaaac caaaaggtct ccgctgacta gggcacatct gacagaagtg gaatcaaggc    720
   tagaaagact ggaacagcta tttctactga tttttcctcg agaagacctt gacatgattt    780
   tgaaaatgga ttctttacag gatataaaag cattgttaga attcccgggt gtcgaccaga    840
   aaaagttcaa taaagtcaga gttgtgagag cactggatgc tgttgctctc ccacagccag    900
   tgggcgttcc aaatgaaagc caagccctaa gccagagatt cactttttca ccaggtcaag    960
   acatacagtt gattccacca ctgatcaacc tgttaatgag cattgaacca gatgtgatct   1020
   atgcaggaca tgacaacaca aaacctgaca cctccagttc tttgctgaca agtcttaatc   1080
   aactaggcga gaggcaactt ctttcagtag tcaagtggtc taaatcattg ccaggttttc   1140
   gaaacttaca tattgatgac cagataactc tcattcagta ttcttggatg agcttaatgg   1200
   tgtttggtct aggatggaga tcctacaaac acgtcagtgg gcagatgctg tattttgcac   1260
   ctgatctaat actaaatgaa cagcggatga aagaatcatc attctattca ttatgcctta   1320
   ccatgtggca gatcccacag gagtttgtca agcttcaagt tagccaagaa gagttcctct   1380
   gtatgaaagt attgttactt cttaatacaa ttcctttgga agggctacga agtcaaaccc   1440
   agtttgagga gatgaggtca agctacatta gagagctcat caaggcaatt ggtttgaggc   1500
   aaaaaggagt tgtgtcgagc tcacagcgtt tctatcaact tacaaaactt cttgataact   1560
   tgcatgatct tgtcaaacaa cttcatctgt actgcttgaa tacatttatc cagtcccggg   1620
   cactgagtgt tgaatttcca gaaatgatgt ctgaagttat tgctgggtcg acgcccatgg   1680
   aattccagta cctgccagat acagacgatc gtcaccggat tgaggagaaa cgtaaaagga   1740
   catatgagac cttcaagagc atcatgaaga agagtccttt cagcggaccc accgaccccc   1800
   ggcctccacc tcgacgcatt gctgtgcctt cccgcagctc agcttctgtc cccaagccag   1860
   caccccagcc ctatcccttt acgtcatccc tgagcaccat caactatgat gagtttccca   1920
   ccatggtgtt tccttctggg cagatcagcc aggcctcggc cttggccccg gcccctcccc   1980
   aagtcctgcc ccaggctcca gcccctgccc ctgctccagc catggtatca gctctggccc   2040
   aggccccagc ccctgtccca gtcctagccc caggccctcc tcaggctgtg gccccacctg   2100
   cccccaagcc cacccaggct ggggaaggaa cgctgtcaga ggccctgctg cagctgcagt   2160
   ttgatgatga agacctgggg gccttgcttg gcaacagcac agacccagct gtgttcacag   2220
   acctggcatc cgtcgacaac tccgagtttc agcagctgct gaaccagggc atacctgtgg   2280
   ccccccacac aactgagccc atgctgatgg agtaccctga ggctataact cgcctagtga   2340
   caggggccca gaggcccccc gacccagctc ctgctccact gggggccccg gggctcccca   2400
   atggcctcct ttcaggagat gaagacttct cctccattgc ggacatggac ttctcagccc   2460
   tgctgagtca gatcagctcc taaggatcct ccggactaga aaagccgaat tctgcaggaa   2520
   ttgggtggca tccctgtgac ccctccccag tgcctctcct ggccctggaa gttgccactc 2580
   cagtgcccac cagccttgtc ctaataaaat taagttgcat cattttgtct gactaggtgt 2640
   ccttctataa tattatgggg tggagggggg tggtatggag caaggggcaa gttgggaaga 2700
   caacctgtag ggctcgaggg ggggcccgaa acccgctgat cagcctcgac tgtgccttct 2760
   agttgccagc catctgttgt ttgcccctcc cccgtgcctt ccttgaccct ggaaggtgcc 2820
   actcccactg tcctttccta ataaaatgag gaaattgcat cgcattgtct gagtaggtgt 2880
   cattctattc tggggggtgg ggtggggcag gacagcaagg gggaggattg ggaagacaat 2940
   agcaaggcat gctggggatg cggtgggctc tatggcttct gaggcggaaa gaaccagctg 3000
   gggctctagg gggtatcccc acgcgccctg tagcggcgca ttaagcgcgg cgggtgtggt 3060
   ggttacgcgc agcgtgaccg ctacacttgc cagcgcccta gcgcccgctc ctttcgcttt 3120
   cttcccttcc tttctcgcca cgttcgccgg ctttccccgt caagctctaa atcgggggct 3180
   ccctttaggg ttccgattta gtgctttacg gcacctcgac cccaaaaaac ttgattaggg 3240
   tgatggttca cgtagtgggc catcgccctg atagacggtt tttcgccctt tgacgttgga 3300
   gtccacgttc tttaatagtg gactcttgtt ccaaactgga acaacactca accctatctc 3360
   ggtctattct tttgatttat aagggatttt gccgatttcg gcctattggt taaaaaatga 3420
   gctgatttaa caaaaattta acgcgaatta attctgtgga atgtgtgtca gttagggtgt 3480
   ggaaagtccc caggctcccc agcaggcaga agtatgcaaa gcatgcatct caattagtca 3540
   gcaaccaggt gtggaaagtc cccaggctcc ccagcaggca gaagtatgca aagcatgcat 3600
   ctcaattagt cagcaaccat agtcccgccc ctaactccgc ccatcccgcc cctaactccg 3660
   cccagttccg cccattctcc gccccatggc tgactaattt tttttattta tgcagaggcc 3720
   gaggccgcct ctgcctctga gctattccag aagtagtgag gaggcttttt tggaggccta 3780
   ggcttttgca aaaagctccc gggagcttgt atatccattt tcggatctga tcagcacgtg 3840
   atgaaaaagc ctgaactcac cgcgacgtct gtcgagaagt ttctgatcga aaagttcgac 3900
   agcgtctccg acctgatgca gctctcggag ggcgaagaat ctcgtgcttt cagcttcgat 3960
   gtaggagggc gtggatatgt cctgcgggta aatagctgcg ccgatggttt ctacaaagat 4020
   cgttatgttt atcggcactt tgcatcggcc gcgctcccga ttccggaagt gcttgacatt 4080
   ggggaattca gcgagagcct gacctattgc atctcccgcc gtgcacaggg tgtcacgttg 4140
   caagacctgc ctgaaaccga actgcccgct gttctgcagc cggtcgcgga ggccatggat 4200
   gcgatcgctg cggccgatct tagccagacg agcgggttcg gcccattcgg accgcaagga 4260
   atcggtcaat acactacatg gcgtgatttc atatgcgcga ttgctgatcc ccatgtgtat 4320
   cactggcaaa ctgtgatgga cgacaccgtc agtgcgtccg tcgcgcaggc tctcgatgag 4380
   ctgatgcttt gggccgagga ctgccccgaa gtccggcacc tcgtgcacgc ggatttcggc 4440
   tccaacaatg tcctgacgga caatggccgc ataacagcgg tcattgactg gagcgaggcg 4500
   atgttcgggg attcccaata cgaggtcgcc aacatcttct tctggaggcc gtggttggct 4560
   tgtatggagc agcagacgcg ctacttcgag cggaggcatc cggagcttgc aggatcgccg 4620
   cggctccggg cgtatatgct ccgcattggt cttgaccaac tctatcagag cttggttgac 4680
   ggcaatttcg atgatgcagc ttgggcgcag ggtcgatgcg acgcaatcgt ccgatccgga 4740
   gccgggactg tcgggcgtac acaaatcgcc cgcagaagcg cggccgtctg gaccgatggc 4800
   tgtgtagaag tactcgccga tagtggaaac cgacgcccca gcactcgtcc gagggcaaag 4860
   gaatagcacg tgctacgaga tttcgattcc accgccgcct tctatgaaag gttgggcttc 4920
   ggaatcgttt tccgggacgc cggctggatg atcctccagc gcggggatct catgctggag 4980
   ttcttcgccc accccaactt gtttattgca gcttataatg gttacaaata aagcaatagc 5040
   atcacaaatt tcacaaataa agcatttttt tcactgcatt ctagttgtgg tttgtccaaa 5100
   ctcatcaatg tatcttatca tgtctgtata ccgtcgacct ctagctagag cttggcgtaa 5160
   tcatggtcat agctgtttcc tgtgtgaaat tgttatccgc tcacaattcc acacaacata 5220
   cgagccggaa gcataaagtg taaagcctgg ggtgcctaat gagtgagcta actcacatta 5280
   attgcgttgc gctcactgcc cgctttccag tcgggaaacc tgtcgtgcca gctgcattaa 5340
   tgaatcggcc aacgcgcggg gagaggcggt ttgcgtattg ggcgctcttc cgcttcctcg 5400
   ctcactgact cgctgcgctc ggtcgttcgg ctgcggcgag cggtatcagc tcactcaaag 5460
   gcggtaatac ggttatccac agaatcaggg gataacgcag gaaagaacat gtgagcaaaa 5520
   ggccagcaaa aggccaggaa ccgtaaaaag gccgcgttgc tggcgttttt ccataggctc 5580
   cgcccccctg acgagcatca caaaaatcga cgctcaagtc agaggtggcg aaacccgaca 5640
   ggactataaa gataccaggc gtttccccct ggaagctccc tcgtgcgctc tcctgttccg 5700
   accctgccgc ttaccggata cctgtccgcc tttctccctt cgggaagcgt ggcgctttct 5760
   catagctcac gctgtaggta tctcagttcg gtgtaggtcg ttcgctccaa gctgggctgt 5820
   gtgcacgaac cccccgttca gcccgaccgc tgcgccttat ccggtaacta tcgtcttgag 5880
   tccaacccgg taagacacga cttatcgcca ctggcagcag ccactggtaa caggattagc 5940
   agagcgaggt atgtaggcgg tgctacagag ttcttgaagt ggtggcctaa ctacggctac 6000
   actagaagaa cagtatttgg tatctgcgct ctgctgaagc cagttacctt cggaaaaaga 6060
   gttggtagct cttgatccgg caaacaaacc accgctggta gcggtggttt ttttgtttgc 6120
   aagcagcaga ttacgcgcag aaaaaaagga tctcaagaag atcctttgat cttttctacg 6180
   gggtctgacg ctcagtggaa cgaaaactca cgttaaggga ttttggtcat gagattatca 6240
   aaaagaatct tcacctagat ccttttaaat taaaaatgaa gttttaatcc aatctaaagt 6300
   atatatgagt aaacttggtc tgacagttac caatgcttaa tcagtgaggc acctatctca 6360
   gcgatctgtc tatttcgttc atccatagtt gcctgactcc ccgtcgtgta gataactacg 6420
   atacgggagg gcttaccatc tggccccagt gctgcaatga taccgcgaga cccacgctca 6480
   ccggctccag atttatcagc aataaaccag ccagccggaa gggccgagcg cagaagtggt 6540
   cctgcaactt tatccgcctc catccagtct attaattgtt gccgggaagc tagagtaagt 6600
   agttcgccag ttaatagttt gcgcaacgtt gttgccattg ctacaggcat cgtggtgtca    6660
   cgctcgtcgt ttggtatggc ttcattcagc tccggttccc aacgatcaag gcgagttaca    6720
   tgatccccca tgttgtgcaa aaaagcggtt agctccttcg gtcctccgat cgttgtcaga    6780
   agtaagttgg ccgcagtgtt atcactcatg gttatggcag cactgcataa ttctcttact    6840
   gtcatgccat ccgtaagatg cttttctgtg actggtgagt actcaaccaa gtcattctga    6900
   gaatagtgta tgcggcgacc gagttgctct tgcccggcgt caatacggga taataccgcg    6960
   ccacatagca gaactttaaa agtgctcatc attggaaaac gttcttcggg gcgaaaactc    7020
   tcaaggatct taccgctgtt gagatccagt tcgatgtaac ccactcgtgc acccaactga    7080
   tcttcagcat cttttacttt caccagcgtt tctgggtgag caaaaacagg aaggcaaaat    7140
   gccgcaaaaa agggaataag ggcgacacgg aaatgttgaa tactcatact cttccttttt    7200
   caatattatt gaagcattta tcagggttat tgtctcatga gcggatacat atttgaatgt    7260
   atttagaaaa ataaacaaat aggggttccg cgcacatttc cccgaaaagt gccacctgac    7320
   gtc                                                                  7323